(19) Europäisches Patentamt
European Patent Office
Office européen des brevets

(11) EP 2 303 353 B1

(12) **EUROPEAN PATENT SPECIFICATION**

(45) Date of publication and mention of the grant of the patent:
**10.01.2018 Bulletin 2018/02**

(21) Application number: **09762175.9**

(22) Date of filing: **11.06.2009**

(51) Int Cl.:
***A61L 31/10*** (2006.01) ***A61L 27/58*** (2006.01)
***A61L 29/14*** (2006.01) ***A61L 31/02*** (2006.01)
***A61L 31/14*** (2006.01) ***A61L 15/44*** (2006.01)
***A61L 15/64*** (2006.01) ***A61L 15/26*** (2006.01)
***A61L 15/42*** (2006.01) ***A61L 27/34*** (2006.01)
***A61L 27/54*** (2006.01) ***A61L 29/08*** (2006.01)
***A61L 29/16*** (2006.01) ***A61L 31/16*** (2006.01)

(86) International application number:
**PCT/IL2009/000581**

(87) International publication number:
**WO 2009/150650 (17.12.2009 Gazette 2009/51)**

(54) **DRUG-ELUTING MEDICAL DEVICES**
ARZNEIMITTEL FREISETZENDE MEDIZINISCHE VORRICHTUNGEN
DISPOSITIFS MÉDICAUX À ÉLUTION DE MÉDICAMENT

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **12.06.2008 US 129234 P**

(43) Date of publication of application:
**06.04.2011 Bulletin 2011/14**

(73) Proprietor: **Ramot at Tel-Aviv University Ltd.**
**61392 Tel Aviv (IL)**

(72) Inventors:
- **ZILBERMAN, Meital**
**69620 Tel-Aviv (IL)**
- **KLOOG, Yoel**
**46585 Herzlia (IL)**
- **KRAITZER, Amir**
**46455 Herzlia (IL)**
- **ELSNER, Jonathan**
**44372 Kfar-Saba (IL)**

(74) Representative: **Dennemeyer & Associates S.A.**
**Postfach 70 04 25**
**81304 München (DE)**

(56) References cited:
**WO-A1-2007/066339** **US-A1- 2004 253 185**
**US-A1- 2005 037 133** **US-A1- 2005 154 451**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 303 353 B1

Printed by Jouve, 75001 PARIS (FR)

## Description

FIELD AND BACKGROUND OF THE INVENTION

The present invention, in some embodiments thereof, relates to the field of material science and, more particularly, but not exclusively, to composite structures and their use as drug-eluting medical devices.

Drug-eluting medical devices have become increasingly in demand in the last decade. Drug-eluting medical devices can be temporary or permanent devices, implantable or topical, and are commonly used in the fields of cardiology and skin treatments.

Organ and tissue failure or loss, such as in burn wounds, trauma wounds, diabetic ulcers and pressure sores, is one of the most frequent and devastating problems in human healthcare. The skin, being the largest organ of the body serving many different functions, still posses some of the most difficult challenges in modern medicine. The moist, warm, and nutritious environment provided by topical wounds, together with a diminished immune function secondary to inadequate wound perfusion, may enable the build-up of physical factors such as devitalized, ischemic, hypoxic, or necrotic tissue and foreign material, all of which provide an ideal environment for bacterial growth. In burns, infection is the major complication after the initial period of shock. It is estimated that about 75 % of the mortality following burn injuries is related to infections and sepsis rather than to osmotic shock and hypovolemia. Drug-eluting wound dressing is one of the most advanced and effective therapeutic solutions to such medical conditions.

Presently known wound dressings are designed to maintain a moist environment to promote healing by preventing cellular dehydration and encouraging collagen synthesis and angiogenesis. Nevertheless, over-restriction of water evaporation from the wound should be avoided as accumulation of fluid under the dressing may cause maceration and sustain infection. Water vapor transmission rate (WVTR) from skin has been found to vary considerably depending on the wound type and healing stage; increasing from 204 grams per square meter per day for normal skin to 278 and as much as 5138 grams per square meter per day, for first degree burns and granulating wounds, respectively. Therefore, the physical and chemical properties of the dressing should be suited to the type of wound and importantly to the degree of exudation from it.

A range of dressing formats based on films, hydrophilic gels and foams are available or have been investigated. These include, for example, OPTSITE® (Smith&Nephew) and BIOCLUSSIVE® (Johnson & Johnson); carboxymethylcellulose-based INTRASITE GEL® (Smith&Nephew) and alginate-based TEGAGEL® (3M); and LYOFOAM® (Mölnlycke Healthcare) and ALLEVYN® (Smith&Nephew).

The partial efficacy of films and foams has encouraged the development of improved wound dressings that provide an antimicrobial effect by eluting germicidal compounds such as iodine (IODOSORB®, Smith&Nephew), chlorohexidime (BIOPATCH®, Johnson & Johnson) or most frequently silver ions (e.g. ACTICOAT® by Smith&Nephew, ACTISORB® by Johnson & Johnson and AQUACELL® by ConvaTec). Such dressings are designed to provide controlled release of the active agent through a slow but sustained release mechanism which helps to avoid toxicity yet ensures delivery of a therapeutic dose to the wound.

Bioresorbable dressings successfully address some of the aforementioned shortcoming, since they do not need to be removed from the wound surface once they have fulfilled their role. Biodegradable film dressings made of lactide-caprolactone copolymers such as TOPKIN® (Biomet) and OPRAFOL® (Lohmann & Rauscher) have been made available. Bioresorbable dressing based on biological materials such as collagen and chitosan have been reported to perform better than conventional and synthetic dressings in accelerating granulation tissue formation and epithelialization. However, controlling the release of antibiotics from these hydrophilic materials is challenging due to the hydrophilic nature of these structures. In most cases, the drug reservoir is depleted in less than two days, resulting in a very short antibacterial effect.

Stents have transformed interventional cardiology in general and coronary angioplasty in particular. Drug-eluting stents (DES), as opposed to bare metal stents (BMS), consist of three parts, namely the stent itself which is an expandable metal alloy framework; a coating layer, typically made of a polymer which can elute a drug into the arterial wall by contact transfer, and the drug which is encapsulated in the polymeric coating and which, for example, inhibits neointimal growth. DES significantly reduce the incidence of in-stent restenosis (ISR), which was once considered a major adverse outcome post percutanous coronary stent implantation.

Currently the most studied and widely used commercially available DES are TAXUS™ by Boston Scientific, which is a paclitaxel eluting stent, and CYPHER™ by Cordis, Johnson & Johnson, which is a sirolimus (rapamycin) eluting stent.

However, drug eluting stents are associated with an increased rate of late stent thrombosis (LST) and hypersensitivity reaction, both of which are life-threatening complications.

Tamai *et al.* [1; 2] were the first researchers to report on immediate and 6 month results after the implantation of a polylactic acid (PLLA) bioresorbable stent in human trials. Bioresorbable PLLA-based stents are taught in numerous publications such as, for example, U.S. Patent Nos. 5,085,629 and 7,169,187 and U.S. Patent Application No.

20030050687 by one of the present inventors.

U.S. Patent Application having Publication No. 20070134305, by one of the present inventors, teaches composite structures, composed of a fibril core and a polymeric coat, which are capable of encapsulating both hydrophobic and hydrophilic bioactive agents while retaining the activity of these agents and favorable mechanical properties of the core fiber. These composite fibers, comprising a coat made of a freeze-dried layer of an emulsion containing a biodegradable polymer and the drug(s), can be used to construct medical devices and disposable articles.

## SUMMARY OF THE INVENTION

The present invention is of composite structures and processes of preparing same, which can be used, for example, as topical and implantable medical devices. Specifically, the present invention is of composite core/coat structures which are designed capable of encapsulating a bioactive agent while retaining the activity of the bioactive agent as well as the desired morphologic and mechanical properties of the core structure. The biodegradable coat of the composite structures is fabricated so as to exhibit a porous microstructure, enabling the containment of a relatively large amount, and a controllable and pre-determined release, of a bioactive agent encapsulated therein.

According to one aspect of embodiments of the present invention there is provided a composite structure which is composed of a device, acting as a core structure, and one or more polymeric porous coat(s) coating at least a part of the device and encapsulating one or more bioactive agent(s), the coat being capable of encapsulating the bioactive agent(s) while retaining the activity of the bioactive agent(s) and/or capable of releasing the bioactive agent(s) in a pre-determined release rate; with the proviso that the device is not a fiber and further with the proviso that when the device is comprised of fibrous elements, the coat is not coating the fibrous elements at the contact point of intercrossing junctions of the fibrous elements in the device, such that the fibrous elements are in contact with each other in each of the junctions.

According to some embodiments of the invention, the device is a medical device.

According to some embodiments of the invention, the medical device is selected from the group consisting of a mesh, a suture mesh, a wound dressing, a stent, a skin patch, a bandage, a suture anchor, a screw, a pin, a tack, a rod, an angioplastic plug, a plate, a clip, a ring, a needle, a tube, a dental implant, an orthopedic implant, a guided tissue matrix, an aortic aneurysm graft device, an atrioventricular shunt, a catheter, a heart valve, a hemodialysis catheter, a bone-fracture healing device, a bone replacement device, a joint replacement device, a tissue regeneration device, a tumor targeting and destruction device, a periodontal device, a hernia repair device, a hemodialysis graft, an indwelling arterial catheter, an indwelling venous catheter, a pacemaker casing, a pacemaker lead, a patent foramen ovale septal closure device, a vascular stent, a tracheal stent, an esophageal stent, a urethral stent, a rectal stent, a stent graft, a synthetic vascular graft, a vascular aneurysm occluder, a vascular clip, a vascular prosthetic filter, a vascular sheath, a drug delivery port and a venous valve.

According to some embodiments of the invention, the polymeric coat is biodegradable.

According to some embodiments of the invention, the device has a mesh structure.

According to some embodiments of the invention, the mesh structure has a form selected from the group consisting of a sheet, a tube, a sphere, a box and a cylinder.

According to some embodiments of the invention, the polymeric coat is characterized by an average pore diameter that ranges from about 1 nm to about 1 mm.

According to other embodiments of the invention, the polymeric coat is characterized by a pore density that ranges from about 5 % of void volume per coat volume to about 95 % of void volume per coat volume.

According to some embodiments of the invention, the thickness of the polymeric coat ranges from about 0.1 $\mu$m to about 2000 $\mu$m.

According to another aspect of embodiments of the present invention there is provided a drug-eluting stent which is composed of a stent device and a polymeric porous coat coating at least a part of the stent device and encapsulating a bioactive agent.

According to some embodiments of the invention, the bioactive agent is a farnesyl derivative as described hereinafter.

According to some embodiments, the farnesyl derivative is farnesylthiosalicylate (FTS).

According to some embodiments, the bioactive agent is paclitaxel.

According to another aspect of embodiments of the present invention there is provided a drug-eluting mesh which is composed of a mesh device and a polymeric porous coat coating at least a part of the mesh device and encapsulating an antimicrobial agent as a bioactive agent.

According to some embodiments of the invention, the antimicrobial agent is selected from the group consisting of gentamicin, ceftazidime and mafenide.

According to some embodiments of the invention, the concentration of the bioactive agent ranges from about 0.1 weight percent to about 10 weight percent of the total weight of the polymeric porous coat.

According to some embodiments of the invention, the polymeric porous coat comprises poly(DL-lactic-co-glycolic acid).

According to another aspect of embodiments of the present invention there is provided a process of preparing a composite structure which includes a device and a porous polymeric coat coating at least a part of the device, the process is effected by contacting the device and an emulsion of an aqueous solution and an organic solution, the organic solution containing one or more polymer(s), to thereby obtain the device having a layer of the emulsion applied on at least a part thereof; and subsequently freeze-drying the device having the layer applied thereon, thereby obtaining the composite structure; with the proviso that the device is not a fiber and further with the proviso that when the device is comprised of fibrous elements, the layer of the emulsion is not applied on the fibrous elements at the contact point of intercrossing junctions of the fibrous elements in the device, such that the fibrous elements are in contact with each other in each of the junctions.

According to yet another aspect of embodiments of the present invention there is provided a process of preparing a composite structure which includes a device and a porous polymeric coat coating at least a part of the device, wherein the coat includes one or more bioactive agent(s) encapsulated therein and/or applied thereon. The process is effected by contacting the device and an emulsion containing an aqueous solution and an organic solution, the organic solution containing one or more polymer(s) and the emulsion further containing the one or more bioactive agent(s) either within the aqueous solution or within the organic solution, to thereby obtain the device having a layer of the emulsion applied on at least a part thereof; and subsequently freeze-drying the device having the layer applied thereon, thereby obtaining the composite structure; with the proviso that the device is not a fiber and further with the proviso that when the device is comprised of fibrous elements, the layer of the emulsion is not applied on the fibrous elements at the contact point of intercrossing junctions of the fibrous elements in the device, such that the fibrous elements are in contact with each other in each of the junctions.

According to some embodiments of the invention, the processes presented herein further includes, prior to the freeze-drying, removing excess of the emulsion, thereby substantially clearing the openings, crevices, grooves and/or crannies in the device.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

As used herein, the singular form "a", "an" and "the" include plural references unless the context clearly dictates otherwise. For example, the term "a compound" or "at least one compound" may include a plurality of compounds, including mixtures thereof.

As used herein the term "about" refers to $\pm$ 10.

The terms "comprises", "comprising", "includes", "including", "having" and their conjugates are used herein as unrestrictive terms. These terms encompass the terms "consisting of" and "consisting essentially of".

The phrase "consisting essentially of" means that the composition or method may include additional ingredients and/or steps, but only if the additional ingredients and/or steps do not materially alter the basic and novel characteristics of the claimed composition or method.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the terms "method" or "process" refer to manners, means, techniques and procedures for accomplishing a given task including those manners, means, techniques and procedures either known to, or readily developed from known manners, means, techniques and procedures by practitioners of the chemical, pharmacological, biological, biochemical and medical arts.

## BRIEF DESCRIPTION OF THE DRAWINGS

Some embodiments of the invention are herein described, by way of example only, with reference to the

accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

FIG. 1 presents a schematic illustration of a drug-eluting stent, an exemplary composite structure according to embodiments of the present invention, wherein mesh-based composite structure **10** is composed of metal stent core structure **12** and porous polymeric coat **14** and whereby porous polymeric coat **14** can encapsulate or otherwise entrap a bioactive agent;

FIG. 2A-B presents a schematic illustration of a core/coat drug-eluting wound dressing, an exemplary composite structure according to embodiments of the present invention, showing the bare pre-coated mesh core as a basic woven mesh of fibers (Figure 2A) and mesh-based composite structure having a porous coat covering the entire mesh-core and filling the openings of the mesh (Figure 2B);

FIGs. 3A-D present SEM fractographs of an exemplary plain-weave composite structure useful as a basic wound dressing according to some embodiments of the present invention, showing a basic unit of the composite structure (FIG. 3A wherein the white bar represents 1 mm), a cross-section of the PDLGA coat matrix which is well adhered to the core suture fibers, forming a coat layer connecting the core fibers (FIG. 3B), and a magnified view of the coat's cross-section having a thickness of about 60 $\mu$m (Figure 3C wherein the white bar represents 50 $\mu$m and Figure 3D wherein the white bar represents 5 $\mu$m);

FIGs. 4A-C present SEM fractographs, showing the effect of a change in an inverted emulsion formulation parameters on the microstructure of the resulting freeze-dried coating matrix containing 5 % w/w ceftazidime and 15 % w/v polymer (50/50 PDLGA, MW 100 KDa), and O:A phase ratio of 6:1, 1 % w/v BSA (formulation ALB1, FIG. 4A), O:A phase ratio of 12:1, 1 % w/v BSA (formulation ALB2, FIG. 4B), and O:A phase ratio of 12:1, 1 % w/v Span 80 (formulation SPA1, FIG. 4C);

FIGs. 5A-B present comparative plots, showing water mass loss as a function of time (FIG. 5A), wherein the results measured from uncovered surface are marked with solid black rectangles, results measured from composite structures made with an emulsion based on 5 % w/w ceftazidime and 15 % w/v polymer (50/50 PDLGA, MW 100 KDa), and O:A phase ratio of 6:1, 1 % w/v BSA (formulation ALB1) are marked with solid blue circles, results measured from composite structures made with emulsion based on 5 % w/w ceftazidime and 15 % w/v polymer (50/50 PDLGA, MW 100 KDa), and O:A phase ratio of 12:1, 1 % w/v BSA (formulation ALB2) are marked with solid red rectangles, results measured from composite structures made with emulsion based on 5 % w/w ceftazidime and 15 % w/v polymer (50/50 PDLGA, MW 100 KDa), and O:A phase ratio of 12:1, 1 % w/v Span 80 (formulation SPA1) are marked with solid green triangles and results measured from dense PDLGA (50/50, MW 100 KDa) film are marked with white rectangle, and showing the water vapor transmission rates (WVTR) for the various wound dressings (FIG. 5B);

FIGs. 6A-B present comparative plots in two time segments, showing the water uptake as a function of time as measured for exemplary composite wound dressing structures coated with two different emulsion formulations, wherein the results measured from the structure coated with an emulsion containing 5 % w/w ceftazidime, 15 % w/v polymer (50/50 PDLGA, MW 100 KDa), O:A phase ratio of 6:1, stabilized with 1 % w/v BSA are marked with solid blue diamonds, and the results measured from the structure coated with a similar emulsion formulation having an O:A modified to 12:1 are marked with solid red rectangles;

FIGs. 7A-D present the results of the mechanical properties studies conducted for composite wound dressing structures, wherein the tensile stress-strain curves for wound dressings immersed in water for 0 weeks (purple line in FIG. 7A), 1 week (red line in FIG. 7A), 2 weeks (green line in FIG. 7A), and 3 weeks (blue line in FIG. 7A), comparing the elastic modulus measured for these samples (FIG. 7B), and the tensile strength (FIG. 7C) and maximal tensile strain (FIG. 7D), as a function of immersion time evaluated from the tensile stress-strain curves, whereas the comparison were made using analysis of variance and significant differences (marked with *);

FIG. 8 presents comparative plots of eluted drug concentrations as a function of time as measured from various drug-eluting mesh-based composite structures prepared according to embodiments of the present invention, wherein the results obtain for gentamicin sulfate-eluting meshes are marked by circles, ceftazidime pentahydrate-eluting meshes are marked by triangles and the results obtain for mafenide acetate-eluting meshes are marked by diamonds, and whereas the formulation parameters of the emulsion used to form the coat of the meshes are 15 % (w/v) polymer in the organic phase, 5 % (w/w) drug concentration, phased ratio of organic to aqueous 6:1 and 1 % albumin as a surfactant;

FIG. 9 presents a SEM micrograph of an farnesylthiosalicylate-eluting stent coated with a porous PDLGA doped with farnesylthiosalicylate, prepared according to embodiments of the present invention, showing that only the struts of the stent are coated, leaving the openings between the struts free of the coat layer;

FIG. 10 presents a SEM micrograph, showing an FTS-eluting stent, and exemplary mesh-based composite structure according to embodiments of the present invention, wherein a fracture was formed intentionally in the coating by freeze-thaw treatment in liquid nitrogen, exposing the stent-coating interface and showing the porous micro-structure of the coat and its adherence to the core structure;

FIG. 11 presents comparative plots of the cumulative drug-release profiles of two PDLGA coated FTS-eluting stents according to embodiments of the present invention, measured over four weeks (28 days) and showing a mean overall release of 53.95±9.73 μg FTS (results are presented as means ± standard deviation);

FIG. 12 presents comparative plots of the normalized accumulated FTS-release profiles of two PDLGA coated FTS-eluting stents according to embodiments of the present invention, showing a mean of 81.38±10.88 % of the total encapsulated FTS released over a period of 28 days and the mean initial burst release of 37.23±7.47 % during the first day of the experiment (results are presented as means ± standard deviation);

FIG. 13 presents a plot of the average molecular weight of the PDLGA coating as a function of time, representing the degradation profile of the porous PDLGA coating, and showing that the rate of degradation during the first 16 days is higher than in the following days (error bars present standard deviation, n=3); and

FIG. 14 presents a photograph of the FTS-eluting stent after 28 days of incubation in PBS medium, showing that the coating is intact and adherent to the stent's struts although massive degradation leading to erosion (weight loss) of the polymer has already occurred over four weeks of exposure to the medium.

## DESCRIPTION OF SPECIFIC EMBODIMENTS OF THE INVENTION

The present invention, in some embodiments thereof, relates to the field of material science and, more particularly, but not exclusively, to composite structures and their use as drug-eluting medical devices.

The principles and operation of the present invention may be better understood with reference to the drawings and accompanying descriptions.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways. Also, it is to be understood that the phraseology and terminology employed herein is for the purpose of description and should not be regarded as limiting.

A recent disclosure in U.S. Patent Application having Publication No. 20070134305, by one of the present invention, teaches a freeze-drying fiber coating technique which is capable of preserving some of the mechanical and spatial characteristics of a hydrophobic/hydrophilic phase emulsion in a solid layer sheathing the fiber. The teachings of U.S. Patent Application having Publication No. 20070134305 are excluded from the scope of the present invention.

While further developing the aforementioned aspects, the present inventors conceived that drug-eluting medical devices can be prepared from preexisting (pre-fabricated) medical devices serving as core structures, which can be dip-coated in an inverted emulsion containing the drug. The coated medical device can be rid of excess emulsion within the opening, holes, grooves and crannies of the original core or not, and can then be freeze-dried so as to obtain a drug-eluting composite structure. This process therefore does not involve the preparation of a coated fibrous structure and the manufacturing of a device from such a coated fibrous structure.

The pre-fabricated core of the drug-eluting composite structure contributes the desired mechanical properties, whereby the porous coat contributes the capacity to contain and controllably release the drug or any other bioactive agent. As presented in the Examples section which follows, the release rate of each bioactive agent from various mesh-based drug-eluting composite structures was monitored and several parameters of the preparation of the coat were examined for their effect on the release profile.

While reducing the present invention to practice, the inventors prepared mesh-based wound dressings composed of pre-weaved polyglyconate fibers, coated with a porous layer loaded with several exemplary antibacterial drugs. These unique biodegradable mesh-based composite structures were designed to be used as bioresorbable burn and/or ulcer dressings, owing to their unique and highly suitable emulsion's composition (formulation) which controls the coat's microstructure, and owing to their favorable drug-release profile.

Modern cardiovascular stents are in principle cylindrical meshes of interconnected struts, made or metal or other materials, which can be coated with a drug-eluting layer to form drug-eluting stents (DES). Thus, in addition, the present inventors have studied and practiced the coating of metal and bioresorbable stents, serving as core structure devices, with a porous emulsion-derived coat loaded with anti-proliferative agents. These DES were tested and proven effective and superior to presently known DES by being capable of encapsulating and releasing a larger amount of drug in a more controlled manner as compared to presently known DES, while not sacrificing and even improving their required mechanical properties.

While further reducing the present invention to practice, the innovative incorporation of the anti-proliferative drug farnesylthiosalicylate (FTS, Salirasib, a Ras antagonist), in a porous coating of a metal stent device (core structure),

derived from freeze drying of inverted emulsions, was achieved successfully. An FTS-eluting stent is expected to overcome the incomplete healing and lack of endothelial coverage associated with current drug eluting stents. The structure, coating degradation profile and FTS release profile are described in the Examples section that follows.

Thus, according to one aspect of the present invention, there is provided a composite structure comprising a device, representing a core structure, and at least one polymeric porous coat coating at least a part of the core structure (the device). The coat of the composite structure presented herein is capable of encapsulating at least one bioactive agent while retaining the activity of the bioactive agent(s) and/or capable of releasing the bioactive agent(s) encapsulated therein in a pre-determined release rate. The composite structure presented herein represents an article, as in an article-of-manufacture, an item or an object.

According to embodiments of the present invention, the core structure can be a medical device or a part of a medical device, such as its casing, which is prefabricated independently. Medical devices, according to the present invention, include, without limitation, a mesh, a suture mesh, a wound dressings, a stent, a skin patch, a bandage, a suture anchor, a screw, a pin, a tack, a rod, an angioplastic plug, a plate, a clip, a ring, a needle, a tube, a dental or orthopedic implant, a guided tissue matrix, an aortic aneurysm graft device, an atrioventricular shunt, a catheter, a heart valve, a hemodialysis catheter, a bone-fracture healing device, a bone replacement device, a joint replacement device, a tissue regeneration device, a tumor targeting and destruction device, a periodontal device, a hernia repair device, a hemodialysis graft, an indwelling arterial catheter, an indwelling venous catheter, a pacemaker casing, a pacemaker lead, a patent foramen ovale septal closure device, a vascular stent, a tracheal stent, an esophageal stent, a urethral stent, a rectal stent, a stent graft, a synthetic vascular graft, a vascular aneurysm occluder, a vascular clip, a vascular prosthetic filter, a vascular sheath, a drug delivery port and a venous valve.

It is noted herein that the composite structures presented herein are not based on stand-alone fibers, namely the core structure or device is not an individual or independent stand-alone fiber. Hence, when the core structure (device) according to some of the present embodiments is composed of or has fibrous elements, the polymeric porous coat does not coat the fibrous elements at the contact point of intercrossing junctions between fibrous elements in the core structure, such that these fibrous elements are in direct physical contact with each other in each of these junctions.

According to some embodiments of the present invention, the core structure is a mesh.

The term "mesh", as used herein, refers to a multidimensional semi-permeable structure that has a large number of closely-spaced holes, which is composed of a plurality of elongated and interconnected elements, such as fibers, strands, struts, spokes, rungs made of a flexible/ductile material, which are arranged in an ordered (matrix, circular, spiral) or random fashion to form a two-dimensional sheet or a three-dimensional object.

According to some of the present embodiments, certain meshes may be composed of fibrous elements which come in direct physical contact with each other at each intercrossing junction constituting the mesh. Thus, a composite structure according to the present embodiments, having a mesh-based device for a core structure is coated with a polymeric porous coat as a whole and does not have a coat at the point of contact (contact point) of intercrossing junctions between the fibrous elements.

A mesh, according to the present embodiments, can be formed by weaving, interlacing, interweaving, knotting, knitting, winding, braiding and/or entangling the elongated elements so they come in contact to form a network of nodes or hubs separated by holes or openings. Alternatively, a mesh can be formed by punching, drilling, cutting or otherwise forming the holes in a sheet of the mesh material.

A three-dimensional mesh is formed by either forming a think sheet, staking several mesh sheets or by bending a mesh sheet into a hollow or tubular object. Exemplary meshes include, without limitation, gauze, a screen, a strainer, a filter, a stent and a wound-dressing. For example, a stent, such as the widely used medical device in angioplasty, bronchoscopy, colonoscopy, esophagogastroduodenoscopy and to treat restenosis and other cardiovascular conditions, is an example of a three-dimensional mesh of struts which are interconnected in a orderly fashion and shaped into a cylindrical tube. Hence, according to embodiments of the present invention, the mesh can take the form or be shaped so as to have a form such as a sheet, a tube, a sphere, a box and a cylinder.

The coating of an entire pre-fabricated core structure such as a mesh as presented herein, is realized in the nodes, junctions, intercrossing, hubs or otherwise the points of contact where individual sub-structural elements meet (referred to herein and encompassed under the phrase "intercrossing junctions"). For example, in the case where the core structure is a mesh, when a mesh is woven from pre-coated fibers, two intercrossing fibrous core elements do not come in contact with each other when they form a junction since they are separated with at least two coat layers sheathing each thereof. In the coated pre-fabricated meshes presented herein, the core elements touch each other via direct physical contact and the entire junction which is formed therebetween is coated as a whole without having a coat material separating the elements. In practice, this feature expresses itself mainly in the way the mesh experiences the gradual degradation of the coat layer. In a mesh which is weaved from pre-coated fibers, the mesh may loosen and even come apart when the coating layers thins and dwindles as a result of its capacity to biodegrade, or in other cases the polymeric coat may swell and cause the element to distance each other causing a deformation of the core structure to some extent, while the coated pre-fabricated meshes do not experience any change due to the erosion or swelling of the coat and

thus the mesh or other similar core structure maintains its structural integrity and stability throughout the process of degradation or swelling of the coat.

Meshes can be formed, woven or otherwise fabricated from fibers made of a natural source such as plants, animal and mineral sources, or be synthetically man-made from naturally occurring and/or synthetic substances. Meshes which are suitable for forming drug-eluting composite structures, according to embodiments of the present invention, can be woven from natural fibers such as cotton, linen, jute, flax, ramie, sisal and hemp, spider silk, sinew, hair, wool and asbestos (the only naturally occurring mineral fiber). Meshes can also be woven from man-made synthetic fibers such as fiberglass, rayon, acetate, modal, cupro, lyocell, nylon, polyester, acrylic polymer fibers, polyacrylonitrile fibers and carbon fiber. Mesh-based core structures can also be formed from biodegradable polymers, as discussed hereinbelow.

The mesh-based core structures can therefore be made of natural or synthetic polymeric materials, elemental materials, metallic substances and any combination thereof. Thus, for example, the mesh core can be a metallic mesh core, made of metals such as, for example, stainless steel and platinum; an elemental mesh core made of carbon and silicon; or a polymeric mesh core made of organic and/or inorganic polymers.

According to some embodiments of the present invention, the core structure is a polymeric core structure, made of a polymeric material. The polymeric core structure can be either degradable or non-degradable (durable), as described in detail hereinbelow.

Thus, according to some embodiments of the present invention, the composite structure includes a polymeric core structure made of biodegradable or non-degradable polymers and/or biodegradable or non-biodegradable co-polymers.

The core structure is the part of the composite structure which bequeaths most of its mechanical and morphologic properties, having been produced by well established techniques which are designed to give the core structure the desired mechanical and morphologic properties.

Meshes used as the core structure of the composite structures can be tailored made so as to provide the composite with the desired properties, selected in accordance with its intended use. The meshes can thus be prepared while controlling the characteristics thereof. Alternatively, commercially or otherwise available meshes can be utilized as the core in the composite structure described herein. Such meshes can be utilized as is or can be subjected to surface treatment prior to use.

Metallic mesh cores, made, for example, from stainless steel are useful in applications that require high mechanical strength and durability. An exemplary application of a composite structure as described herein, which has a stainless steel mesh core, is a stent. A bare metal stent is one example of a commercially available mesh. Bare metal stents can serve as the core according to the embodiments of the present invention where high resilience and springiness are desired.

A mesh-based composite structure as presented herein is therefore composed of two basic elements: a mesh-based core structure and a (single or multiple) coat, whereby the structure as a whole adopts the shape of the mesh-based core structure.

Figure 1 presents a schematic illustration of a drug-eluting stent, an exemplary composite structure according to embodiments of the present invention. As can be seen in Figure 1, mesh-based composite structure **10** is composed of metal stent core structure **12** and porous polymeric coat **14;** whereby porous polymeric coat **14** can encapsulate or otherwise entrap a bioactive agent.

Figure 2 presents a schematic illustration of a drug-eluting wound dressing, another exemplary composite structure according to embodiments of the present invention. As can be seen in Figure 2A, the mesh core is a basic woven mesh of fibers, suitable for use as wound dressing mesh. As can be seen in Figure 2B, the porous coat can cover the entire mesh and fill the openings of the mesh.

The incorporation of the mesh-based core structure into the composite structures presented herein does not weaken or otherwise adversely affect the properties of the mesh.

As mentioned above, in some embodiments of the present invention, the mesh-based core structure is a polymeric mesh core structure. As is further mentioned hereinabove, the coat coating the mesh core structure is also a polymeric coat.

The term "polymer", as used herein, encompasses organic and inorganic polymer and further encompasses one or more of a polymer, a copolymer or a mixture thereof (a blend).

While any polymer, copolymer or a mixture of polymers and/or copolymers can be used for producing the core and coat of the structures described herein, according to some embodiments of the present invention, the coat is made of a biodegradable polymer.

The term "biodegradable", "bioresorbable" and "bioabsorbable", as used interchangeably in the context of the present invention, describes a material which can decompose under physiological and/or environmental conditions into breakdown products. Such physiological and/or environmental conditions include, for example, hydrolysis (decomposition via hydrolytic cleavage), enzymatic catalysis (enzymatic degradation), and mechanical interactions. This term typically refers to substances that decompose under these conditions such that 50 weight percents of the substance decompose within a time period shorter than one year.

The terms "bioresorbable" and "bioabsorbable" further describe a substance that decomposes under physiological conditions to break down to products that undergo bioresorption into the host-organism, namely, become metabolites of the biochemical systems of the host-organism.

It is noted herein that there are some biodegradable polymers that degrade very slowly in relative terms. For example, polycaprolactone-based structural elements exhibit noticeable degradation only after a period of 4-5 years. The terms "bioresorbable" and "bioabsorbable" therefore encompass substances the biodegrade within a time period that ranges from a few hours and a few years, including a few days and a few months.

The incorporation of a biodegradable coat in the composite structure described herein results, for example, in the release of bioactive agents that are potentially encapsulated in the coat when the latter is exposed to physiological conditions.

Further according to some embodiments of the present invention, the core can be either biodegradable or non-degradable. Thus, the coating of the core structure can be made from a stable non-degradable polymer. In such a case the drug will be released from the porous coating according to diffusion controlled processes through a porous medium and in some cases swelling of the host polymer in water will further augment the drug-release profile although no degradation of the host polymer will be involved is that process.

As used herein, the term "non-degradable" describes a substance which does not undergo degradation under physiological and/or environmental conditions. This term typically refers to substances which decompose under these conditions such that more than 50 percents do not decompose within at least 1 year, within 2 years, 3 years, 4 years, and up to 10 years and even 20 or 50 years.

Structures comprising a non-degradable core are useful, for example, in applications which require at least part of the scaffold to be tenable.

An exemplary non-degradable polymer suitable for use as core structure in the context of the present invention is nylon. A non-biodegradable core can be prepared from pre-treated nylon suture meshes and be coated with a porous polymeric coat, while maintaining the physical, chemical and mechanical properties nylon mesh.

Structures comprising a biodegradable core are desired in applications where degradation of the whole structure overtime is desired.

In embodiments where both the core and the coat are biodegradable, each is composed of a first and second biodegradable polymer, respectively.

Exemplary biodegradable polymers according to the present embodiments are non-toxic and benign polymers. Some biodegradable polymers are bioresorbable polymers which decompose into non-toxic and benign breakdown products that are absorbed in the biochemical systems of the subject.

Non-limiting examples of biodegradable polymers which are suitable for use as the first and second biodegradable polymers composing the core and coat of the composite structure described herein, respectively, include homopolymers and co-polymers such as aliphatic polyesters made of glycolide (glycolic acid), lactide (lactic acid), caprolactone, p-dioxanone, trimethylene carbonate, hydroxybutyrate, hydroxyvalerate, polypeptide made of natural and modified amino acids, polyethers made of natural and modified saccharides, polydepsipeptide, biodegradable nylon copolyamides, polydihydropyrans, polyphosphazenes, poly(ortho-esters), poly(cyano acrylates), polyanhydrides and any combination thereof.

According to an exemplary embodiment of the present invention, the biodegradable polymer is an aliphatic polyester such as, for example, poly(glycolic acid), poly(lactic acid), polydioxanone (PDS), poly(alkylene succinate), poly(hydroxybutyrate), poly(butylene diglycolate), poly(epsilon-caprolactone) and a co-polymer, a blend and a mixture thereof.

Exemplary aliphatic polyesters that were found suitable for use in the context of the present invention include poly(L-lactic acid), poly(glycolic acid) and/or co-polymers thereof such as poly(DL-lactic-co-glycolic acid).

According to an embodiment of the present invention, the polymeric coat is made of poly(DL-lactic-co-glycolic acid). An exemplary poly(DL-lactic-co-glycolic acid) that was found suitable for use in this context of the present invention has a ratio of DL-lactic acid to glycolic acid of 75 weight percentages to 25 weight percentages respectively. Manipulating the lactic acid:glycolic acid ratio in the co-polymer, however, can affect the chemical and physical properties of the coat. Thus, for example, using polymers with a higher content of glycolic acid (such as for example a 50:50. lactic acid:glycolic acid ratio) results in a polymeric porous coat with smaller pore size, while using polymers with higher contents of lactic acid (such as for example, poly(lactic acid) results in a polymeric porous coat with larger pore size.

The polymeric coat, according to the present embodiments, can cover the core structure either partially or, alternatively, entirely by forming a layer on the core structure’s surface. The layer can be a continuous layer along one side of the core structure, a multitude of discontinuing patches, and/or a combination thereof, or form a complete coat which envelops the core structure.

According to some embodiments of the present invention, the coat covers the core structure without filling or

otherwise obstructing the opening, crevices, grooves and/or crannies in the core structure, thus maintaining its complex and detailed, and in some cases its semi-permeable, morphology and other mechanical properties thereof. However, in other embodiments the coat may fill some or all the voids and openings of the core structure.

The thickness of the coat can be tailored so as to suit any specific application for which the composite structures are used for. For example, for long-range temporal drug delivery, a large reservoir of the drug is required, and hence a relatively thick coat is useful and desired in many applications. A relatively thick coat is also required to encapsulate large bioactive agents such as virus-shells and cells, while the entrapment of relatively small drug molecules which are needed in small locally-distributed amounts may suffice with a relatively thin coat. Therefore, the thickness of the coat, layered on the core structure according to the present embodiments, can range from about 10 $\mu$m to about 2000 microns and in certain cases can be even up to 1 cm.

The choice of a certain thickness of the coat may further depend on the core thickness, the core-coat ratio in the structure and the desired thickness of the structure as a whole.

According to an exemplary embodiment of the present invention, the coat has a porous microstructure. As used herein, the term "porous" refers to a consistency of a solid material, such as foam, a spongy solid material or a frothy mass of bubbles embedded and randomly dispersed within a solid matter.

A porous polymeric coat is highly beneficial since it allows a controlled release of agents encapsulated therein. In the context of the present invention, the porosity or porousness (the coat's microstructure) can be regarded as a combination of three criteria, namely the density of the pores, the average pore size (diameter), and the tortuosity which accounts for how many of the pores are interconnected so as to form a continuous void inside the solid part of the coat. The tortuosity is correlated to the pore density and the average pore size since the inter-connectivity or discreteness of the pores depends on both the size and density thereof.

The coat of the composite structure of the present invention is designed capable of encapsulating, entrapping or enveloping one or more bioactive agents therein.

Specifically, the composite structure according to the present embodiments is designed capable of encapsulating one or more bioactive agent(s) within the (voids or pores) of the coat. Alternatively or in addition, the bioactive agent(s) can be attached to the inner surface of the coat, applied on the outer surface of the coat and/or encapsulated within the polymeric coat itself.

According to embodiments of the present invention, the core structure can be coated with more than one layer, each having different composition and thus exhibit different properties such as biodegradability, density, porosity and other mechanical characteristics, and contain a different bioactive agent.

Thus, each of the composite structures of the present embodiments can further comprise one or more bioactive agents. The bioactive agent can be encapsulated within or attached to or on the polymeric coat described herein and/or can be encapsulated in the core structure described herein.

Furthermore, the composite structure according to the present embodiments is designed such that the encapsulation of the bioactive agent is performed while retaining at least a part, most or all of the activity of the bioactive agent(s). Thus, these agents can exert their biological activity and/or therapeutic effect once the bioactive agent(s) is released to the physiological environment, as a result of the biodegradation of the coat and/or the bond used for attaching it to the coat.

The release (elution) process depends on and is controlled by the degradation process, which in turn is carried out enzymatically, chemically or via other metabolic reactions in the physiological environment both *in vivo* and *in vitro.* First to degrade would be the outer surface of the composite structure, and in most cases, where the coat forms an entire envelope, the coat would be first to degrade while being exposed to the physiological environment. As the coat is degraded and consumed and the pores are gradually exposed to the physiological environment, the bioactive agent(s) encapsulated in the coat is released.

A release process of bioactive agents from the coat can therefore be controlled by manipulating the composition of the biodegradable polymer composing the coat, the size, length and diameter of the composite structure, the thickness of the coat, the size and density of the pores, the amount and physicochemical properties of the bioactive agent(s) encapsulated within or applied on the coat during the preparation process of the composite structure. As aforementioned, and without being bound to any particular theory, drug molecules can be released also from a non-degradable host polymer, which can undergo some "swelling" in aqueous media or not.

Some of these attributes were tested for their effect on the release profile of several exemplary bioactive agents, namely gentamicin, ceftazidime, mafenide acetate, paclitaxel and farnesylthiosalicylate, from exemplary composite structures, as is demonstrated and exemplified in the Examples section that follows and is further detailed hereinbelow.

The biodegradation of the coat and/or the core may further be controlled by the addition of agents which can control and modify the biodegradation rate of the polymer composing the core and/or coat. Hence, according to embodiments of the present invention, the biodegradable coat and/or the biodegradable core structure further include a biodegradation promoting agent.

A biodegradation promoting agent accelerates the chemical and/or biochemical degradation processes by

providing the required chemical conditions such as pH, ionic-strength, highly-active and readily activated species and enzymatic co-factors. Non-limiting examples of biodegradation promoting agents include cellulose phosphates, starch phosphates, calcium secondary phosphates, calcium tertiary phosphates and calcium phosphate hydroxide.

As used herein, the phrase "bioactive agent" describes a molecule, compound, complex, adduct and/or composite that exerts one or more biological and/or pharmaceutical activities. The bioactive agent can thus be used, for example, to promote wound healing, tissue regeneration, tumor eradication, and/or to prevent, ameliorate or treat various medical conditions.

"Bioactive agents", "pharmaceutically active agents", "pharmaceutically active materials", "therapeutic active agents", "biologically active agents", "therapeutic agents", "drugs" and other related terms are used interchangeably herein and include, for example, genetic therapeutic agents, non-genetic therapeutic agents and cells. Bioactive agents useful in accordance with the present invention may be used singly or in combination. The term "bioactive agent" in the context of the present invention also includes radioactive materials which can serve for radiotherapy, where such materials are utilized for destroying harmful tissues such as tumors in the local area, or to inhibit growth of healthy tissues, such as in current stent applications; or as biomarkers for use in nuclear medicine and radioimaging.

The bioactive agent can be a hydrophilic bioactive agent or a hydrophobic bioactive agent.

The term "hydrophilic", as used herein, describes a trait of a molecule or part of a molecule which renders the molecule dissolvable, at least in part, in water, aqueous solutions and/or other polar solvents. The phrase "at least in part" means that the substance is either completely dissolvable in such solvents or reaches its maximal saturation concentration in water, aqueous solutions and/or other polar solvents, while the remainder of the substance is in the form of a suspension of small solid particles in the solvent. Hydrophilic agents are therefore typically water-soluble agents, in which the dissolvability of the molecule in water, aqueous solutions and polar solvents is higher than its dissolvability in oils, organic solvents and other non-polar solvents. The term "hydrophilic", as used and defined herein, also encompasses amphiphilic or amphiphatic agents, which are characterized by a part of the molecule that is hydrophilic and hence renders the molecule dissolvable, at least to some extent, in water and aqueous solutions.

The terms "amphiphilic" or "amphiphatic", as used herein, refer to a trait of a molecule having both hydrophilic and hydrophobic nature, namely a polar region that can be either ionic, or non-ionic, and a non-polar region.

Exemplary hydrophilic substances include, without limitation, compounds comprising one or more charged or polar groups such as one or more carboxyl groups (e.g., organic acids), one or more hydroxyl groups (e.g., alcohols), one or more amino groups (e.g., primary, secondary, tertiary and quaternary amines), and any combination thereof. Such groups are present, for example, in peptides and saccharides and in many other naturally occurring and synthetic substances.

Amphiphilic substances also comprise, alongside with charged or polar groups, also non-polar moieties such as those exhibited in hydrophobic substances, as these are defined hereinbelow. Exemplary types of amphiphilic molecules include, without limitation, anionic molecules (such as sodium dodecyl sulfate), cationic molecules (such as benzalkonium chloride), zwitterionic molecules (such as cocamidopropyl betaine) and non-ionic molecules (such as octanol).

Representative examples of hydrophilic and/or of amphiphilic bioactive agents that can be beneficially incorporated in the coat described herein include, without limitation, amino acids and peptide- and protein-based substances such as cytokines, chemokines, chemo-attractants, chemo-repellants, agonists, antagonists, antibodies, antigens, enzymes, co-factors, growth factors, haptens, hormones, and toxins; nucleotide-based substances such as DNA, RNA, oligonucleotides, labeled oligonucleotides, nucleic acid constructs, and antisenses; saccharides, polysaccharides, phospholipids, glycolipids, viruses and cells, as well as hydrophilic or amphiphatic radioisotopes, radiopharmaceuticals, steroids, non-steroidal anti-inflammatory agents, steroidal anti-inflammatory drugs, vitamins, angiogenesis-promoters, drugs, antihistamines, antibiotics, antidepressants, anti-hypertensive agents, anti-inflammatory agents, antioxidants, anti-proliferative agents, anti-viral agents, chemotherapeutic agents, co-factors, cholesterol, fatty acids, bile acids, saponins, hormones, inhibitors and ligands, and any combination thereof.

The term "hydrophobic", as used herein, refers to a trait of a molecule or part of a molecule which is non-polar and is therefore immiscible with charged and polar molecules, and has a substantially higher dissolvability in non-polar solvents as compared with their dissolvability in water and other polar solvents. The term "dissolvability" refers to either complete dissolution of the substance in these solvents or to cases where the substance reaches its maximal saturation concentration in non-polar solvents, and the remainder of the substance is in the form of a suspension of small solid particles in the solvent. When in water, hydrophobic molecules often cluster together to form lumps, agglomerates, aggregates or layers on one of the water surfaces (such as bottom or top). Exemplary hydrophobic substances include, without limitation, substances comprising one or more alkyl groups, such as oils and fats, or one or more aromatic groups, such as polyaromatic compounds.

Representative examples of hydrophobic bioactive agents that can be beneficially incorporated in the coat described herein include, without limitation drugs, anti-coagulants, statins, hormones, steroids, lipids, antibiotics, antigens, antidepressants, anti-hypertensive agents, anti-inflammatory agents, antioxidants, anti-proliferative agents, anti-viral agents, chemotherapeutic agents, haptens, inhibitors, ligands, radioisotopes, radiopharmaceuticals, toxins and any combination thereof.

Each of the hydrophilic and hydrophobic bioactive agents described herein can be a macro-biomolecule or a small, organic molecule.

The term "macro-biomolecules" as used herein, refers to a polymeric biochemical substance, or biopolymers, that occur naturally in living organisms. Polymeric macro-biomolecules are primarily organic compounds, namely they consist primarily of carbon and hydrogen, along with nitrogen, oxygen, phosphorus and sulfur, while other elements can be incorporated therein but at a lower rate of occurrence. Amino and nucleic acids are some of the most important building blocks of polymeric macro-biomolecules, therefore macro-biomolecules are typically comprised of one or more chains of polymerized amino acids, polymerized nucleic acids, polymerized saccharides, polymerized lipids and combinations thereof. Macromolecules may comprise a complex of several macromolecular subunits which may be covalently or non-covalently attached to one another. Hence, a ribosome, a cell organelle and even an intact virus can be regarded as a macro-biomolecule.

A macro-biomolecule, as used herein, has a molecular weight higher than 1000 dalton (Da), and can be higher than 3000 Da, higher than 5000 Da, higher than 10 kDa and even higher than 50 KDa.

Representative examples of macro-biomolecules, which can be beneficially incorporated in the coat described herein include, without limitation, peptides, polypeptides, proteins, enzymes, antibodies, oligonucleotides and labeled oligonucleotides, nucleic acid constructs, DNA, RNA, antisense, polysaccharides, viruses and any combination thereof, as well as cells, including intact cells or other subcellular components and cell fragments.

As used herein, the phrase "small organic molecule" or "small organic compound" refers to small compounds which consist primarily of carbon and hydrogen, along with nitrogen, oxygen, phosphorus and sulfur and other elements at a lower rate of occurrence. In the context of the present invention, the term "small" with respect to a compound, agent or molecule, refers to a molecular weight lower than about 1000 grams per mole. Hence, a small organic molecule has a molecular weight lower than 1000 Da, lower than 500 Da, lower than 300 Da, or lower than 100 Da.

One class of bioactive agents is the class of therapeutic agents that promote angiogenesis, which can be encapsulated in the coat of the composite structures useful for tissue regeneration and wound dressings. The successful regeneration of new tissue requires the establishment of a vascular network. The induction of angiogenesis is mediated by a variety of factors, any of which may be used in conjunction with the present invention (Folkman and Klagsbrun, 1987, and references cited therein).

Another class of bioactive agents which can be incorporated into the coat of the composite structures of the present embodiments, especially in certain embodiments which involve tissue regeneration, implantable devices and healing are cytokines, chemokines and related factors. Control over these agents can translate into a successful medical procedure when the immune system plays a key role.

Bioactive agents which can be beneficially incorporated into the coat of the composite structures of the present embodiments also include both natural or synthetic polymeric (macro-biomolecules, for example, proteins, enzymes) and non-polymeric (small molecule therapeutics) natural or synthetic agents.

Additional bioactive agents which can be beneficially incorporated into the coat of the composite structures of the present embodiments include anti-proliferative agents, cytotoxic factors or cell cycle inhibitors, including CD inhibitors, such as p53, thymidine kinase ("TK") and other agents useful for interfering with cell proliferation.

Bioactive agents that inhibit cell proliferation and/or angiogeriesis (antiproliferative drugs) are particularly useful in drug-eluting stents, and include paclitaxel (TAXOL®), sirolimus (rapamycin) and farnesylthiosalicylate (FTS, salirasib), fluoro-FTS, everolimus (RAD-001) and zotarolimus. The encapsulation of two exemplary antiproliferative drugs, farnesylthiosalicylate and paclitaxel, in a porous coat of stents and fibers, is demonstrated in the Examples section that follows.

Additional bioactive agents that may be encapsulated beneficially into the coating of composite structures of the present embodiments include FTS-methyl ester (FTS-ME), FTS-methoxy-methylene ester (FTS-MOME), and FTS-amide (FTS-A) [Goldberg, L. et al., J. Med. Chem. (2009), 52(1), pp. 197-205], or 5-fluoro-FTS [Marciano, D. et al., J. Med. Chem., (1995), 38(8), pp. 1267-1272]. These antiproliferative agents can be encapsulated by a porous coat of composite structures of medical devices such as, for examples, stents, implantable a tumor targeting and destruction device and topical devices such as meshes and patches. These stents and other tumor targeting and destruction devices which can elute one or more

antiproliferative agents and drugs can be implanted or otherwise placed near or on a tumor site, or near or on a tumor site post its surgical removal.

Additional bioactive agents which can be beneficially incorporated into the coat of the composite structures of the present embodiments include gene delivery agents, which may be either endogenously or exogenously controlled, the family of bone morphogenic proteins ("BMP's"), cell survival molecules such as Akt, insulin-like growth factor 1, NF-kB decoys, 1-kB, Madh6, Smad6 and Apo A-1, viral and non-viral vectors and chemotherapeutic agents. Non-limiting examples of chemotherapeutic agents include amino containing chemotherapeutic agents such as daunorubicin, doxorubicin, N-(5,5-diacetoxypentyl)doxorubicin, anthracycline, mitomycin C, mitomycin A, 9-amino camptothecin, aminopertin, antinomycin, $N^8$-acetyl spermidine, 1-(2-chloroethyl)-1,2-dimethanesulfonyl hydrazine, bleomycin, tallysomucin, and derivatives thereof; hydroxy containing chemotherapeutic agents such as etoposide, camptothecin, irinotecaan, topotecan, 9-amino camptothecin, paclitaxel, docetaxel, esperamycin, 1,8-dihydroxy-bicyclo[7.3.1]trideca-4-ene-2,6-diyne-13-one, anguidine, morpholino-doxorubicin, vincristine and vinblastine, and derivatives thereof, sulfhydril containing chemotherapeutic agents and carboxyl containing chemotherapeutic agents.

Additional bioactive agents which can be beneficially incorporated into the coat of the composite structures of the present embodiments include antibiotic agents. Non-limiting examples of antibiotic agents include gentamicin, ceftazidime, mafenide benzoyl peroxide, octopirox, erythromycin, zinc, silver, tetracyclin, triclosan, azelaic acid and its derivatives, phenoxy ethanol and phenoxy proponol, ethylacetate, clindamycin and meclocycline; sebostats such as flavinoids; alpha and beta hydroxy acids; polydiallyldimethylammonium chloride and bile salts such as scymnol sulfate and its derivatives, deoxycholate and cholate. Three exemplary antibiotic agents, gentamicin, ceftazidime and mafenide, were used to demonstrate the efficiency of the composite structure presented herein, as presented in the Examples section that follows.

Additional bioactive agents which can be beneficially incorporated into the coat of the composite structures of the present embodiments include cells of human origin (autologous or allogeneic), including stem cells, or from an animal source (xenogeneic), which can be genetically engineered if desired to deliver proteins of interest.

As discussed hereinabove, the porosity (microstructure) of the coat can determine the release profile of the bioactive agent therefrom, and it is defined by the average pore size (diameter) and the density thereof, which also reflect the level of inter-connectivity of the pores.

In general, according to some embodiments of the present invention, the porosity of the coat is characterized by an average pore diameter that can range from 0.001 μm (1 nm) to 1000 μm (1 mm), and a pore density that can range from about 5 % void volume per coat volume to about 95 % void volume per coat volume, or from about 8 % void volume per coat volume to about 95 % void volume per coat volume, or from about 10 % void volume per coat volume to about 95 % void volume per coat volume, or from about 10 % void volume per coat volume to about 90 % void volume per coat volume, or from about 60 % void volume per coat volume to about 95 % void volume per coat volume, including any integer within the above-indicated ranges (e.g., 5, 6, 7, 8, 9, 10, 11, 12, 13, 14, 15, 16, 17, 18, 19, 20 %, etc., up to 95 %).

Several factors affect the resulting pore size and density, including the nature of the bioactive agent which is incorporated into the coat, and the process of preparing the coat.

The coat's microstructure strongly affects the rate of release of the incorporated bioactive agent. According to the present embodiments, the average pore diameter and density in the porous coat can be finely controlled so as to enable a particularly desirable release profile of the encapsulated agent which is suitable for a particular application. In turn, the nature of the bioactive agent, namely its capacity to dissolve in water and other aqueous solutions, affects the coat's microstructure.

Furthermore, without being bound to any particular theory, stemming from the process of preparing the coat, presented hereinbelow, it is assumed that a hydrophobic bioactive agent will be incorporated into the solid walls of the coat or near the surface (inner walls) thereof, while hydrophilic and amphiphilic agents will be incorporated in or on the inner walls of the pores. Hence, when introduced into a physiological medium, which is substantially aqueous, a hydrophilic agent will be exposed to the solvent (water) as soon as the solvent enters the void constituting a pore, and therefore will be released immediately upon the exposure of the pore to the physiological medium. On the other hand, a hydrophobic bioactive agent with resides inside the solid polymeric walls of the coat will be released according to the surface area of the solid polymer and according to other diffusion controlled and swelling parameters, and will be affected by the rate of degradation of the solid polymer when using a degradable polymer.

When attempting to design the release profile of a bioactive agent form a composite structure as presented herein, one has to consider the desirable burst-rate which takes place as soon as the composite structure is exposed to the host medium, and the diffusion-controlled rate of release which follows the initial burst. These stages of release can be controlled by the porosity of the coat which dictates the surface area exposed to the medium and the detailed microstructure of the coat.

For example, a hydrophilic bioactive agent, which is assumed to be incorporated on the inner walls of the pores, will be all released as soon as the coat is exposed to an aqueous media in case the pores are substantially interconnected. In order to lower the extent of this burst, and allow the agent to be released in a more prolonged and steady rate, the pores should be discrete so the inner void of each is exposed to the medium only upon degradation of its solid polymer wall.

On the other hand, a burst release of hydrophobic bioactive agent, which resides within the solid polymer part of the coat, will be possible if a large surface area of the polymer is exposed simultaneously to the medium, and therefore the porosity of a composite structure which incorporates a hydrophobic agent exhibits interconnected pores so to allow the medium to penetrate deep into the coat and bring about its degradation more effectively.

Regardless of its water-solubility, a relatively large bioactive agent, such as a virus, an organelle or a cell would require a suitable pore size to fit its size. Thus, in the case of a large bioactive agent, the porosity will be characterized

by a large pore size.

Thus, for example, porous coats designed to encapsulate or encapsulating a hydrophilic/amphiphilic (water-soluble) bioactive agent, have an average pore diameter ranges from about 1 nm to about 50 μm, a density ranges from about 10 % of void volume per coat volume to about 90 % of void volume per coat volume, and /or discrete pores.

Porous coats designed to encapsulate or encapsulating a hydrophobic (water-insoluble) bioactive agent, have an average pore diameter ranges from about 1 nm to about 200 μm, a density that ranges from about 50 % of void volume per coat volume to about 95 % of void volume per coat volume, and/or interconnected pores.

In cases where the encapsulated agent comprises large macro-biomolecules, assemblies thereof, organelles or intact cells, larger pores, having an average pore diameter that ranges from about 50 μm to about 500 μm and higher are useful.

As detailed hereinbelow and is further demonstrated in the Examples section that follows, a suitable porosity can be adjusted to almost any bioactive agent by modifying certain parameters in the process of preparing the composite structures presented herein and by the use of additional agents and other mechanical and kinetic factors which contribute to the final microstructure of the coat, utilizing this flexibility towards a wide range of therapeutic and other applications.

One group of additional agents which may contribute to the final microstructure of the coat includes surfactants or surface active agents, as these are defined hereinbelow. As demonstrated in the examples section that follows, the addition of a surfactant at the preparation stage of the coat material affects the porosity thereof and in some cases is essential to the formation of the coat. The requirement of a surfactant is strongly associated with the nature of the bioactive agent, namely its hydrophobicity or lack thereof. A hydrophobic bioactive agent and a hydrophilic bioactive agent may not contribute to the stability of the coat's precursor, while an amphiphilic bioactive agent, which may act as a surfactant in most cases, will render the use of an additional surface active agent unnecessary.

The coat can further include, in addition to the bioactive agent, additional agents that may improve the performance of the bioactive agent. These include, for example, penetration enhancers, humectants, chelating agents, preservatives, occlusive agents, emollients, permeation enhancers, and anti-irritants. These agents can be encapsulated within the pores of a porous coat or can be doped within the polymer forming the coat.

The amount of the bioactive agents that is loaded in the composite structure is selected sufficient to exert the desired therapeutic or biological effect. The effective amount of a bioactive agent therefore depends on the particular agent being used and can further depend on the desired application of the resulting structure. Thus, for example, in cases where the bioactive agent is a growth hormone, minute amounts of the agent are required so as to exert effective therapy. In cases where the bioactive agent is a protein or a peptide, medium range amounts of the agent are required. In cases where the bioactive agent is a metabolite having a high metabolic turnover rate or a chemical drugs, larger amounts of the bioactive agent are typically required.

Therefore, the amount of the bioactive agent in the composite structures can range from about 0.00001 weight percentage to about 50 weight percentages of the amount of the total weight of the coat, and ranges from about 0.1 weight percentage to about 30 weight percentages of the amount of the total weight of the coat, or from about 1 weight percentage to about 20 weight percentages or from about 1 weight percentage to about 10 weight percentages of the total weight of the coat, in cases where the bioactive agent is a biomolecules such as a peptide.

As indicated hereinabove, for bioactive agents such as growth factors, an amount in the composite structures of from about 0.00001 to about 0.0001 percents of the total weight of the coat is sufficient to exert the desired activity, whereby for bioactive agents such as, for example, synthetic drugs, an amount in the composite structures of from about 1 to about 30 percents of the total weight of the coat is useful.

The amount of the bioactive agent further affects the rate of release thereof, particularly in cases where the bioactive agent is encapsulated within the pore voids (a hydrophilic/amphiphatic agent), due to diffusion-related factors. Hence, the amount of the bioactive agent can be further manipulated in accordance with the desired release rate thereof.

According to some embodiments of the present invention, the composite structure is a drug-eluting stent. In some embodiments the stent device, serving as a core structure, is a bare metal stent (BMS), made from, for example, stainless steel. Such a BMS can be coated with a porous coat made of poly(DL-lactic-co-glycolic acid), as detailed herein.

According to some embodiments, the bioactive agents which can be beneficially used in the context of a drug-eluting stent (DES) as presented herein, is a farnesyl derivative or analog. Many farnesyl derivatives are well known in the art, and some are know to have anti-proliferative activity which is highly beneficial in the context of a DES.

U.S. Patent No. 5,705,528, by one of the present inventors, teaches farnesyl derivatives which are inhibitors of the prenylated protein thyltransferase enzyme, which can be used beneficially as anti-cancer/anti-proliferative drugs. Some of the farnesyl derivatives, taught in U.S. Patent No. 5,705,528, have the following Formula I:

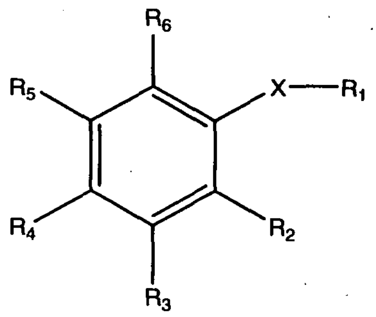

Formula I

wherein:

$R_1$ is selected from the group consisting of farnesyl, geranyl or geranyl-geranyl;
$R_2$ is selected from the group consisting of hydrogen, -C=N, $-COOR_7$, $-SO_3R_7$,$-CONR_7R_8$ and $SO_2NR_7R_8$, -COOM and $-SO_3M$;
$R_7$ and $R_8$ are each independently selected from the group consisting of hydrogen, alkyl and alkenyl;
M is a cation;
$R_3$, $R_4$, $R_5$ and $R_6$ are each independently selected from the group consisting of hydrogen, carboxyl, alkyl, alkenyl, aminoalkyl, nitroalkyl, nitro, halo, amino, mono- or di-alkylamino, mercapto, mercaptoalkyl, azido, or thiocyanato;
X is selected from the group consisting of O, S, SO, $SO_2$, NH or Se.

Farnesyl derivatives which are beneficial in the context of a DES according to the present embodiments, include:

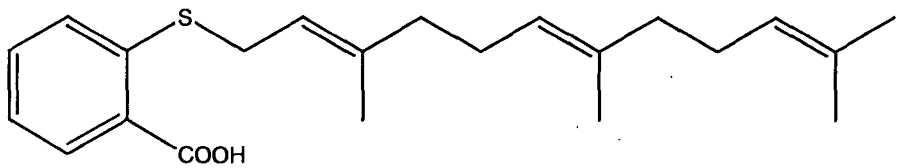

2-((2E,6E)-3,7,11-trimethyldodeca-2,6,10-trienylthio)benzoic acid; and

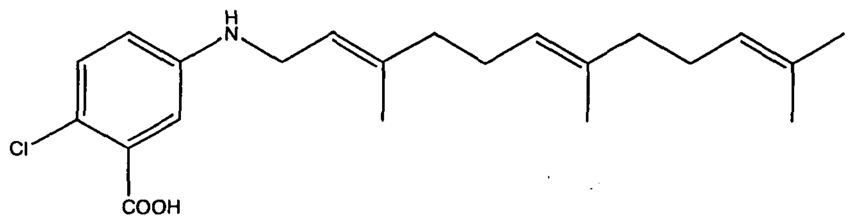

5-((2E,6E)-3,7,11-trimethyldodeca-2,6,10-trienylamino)-2-chlorobenzoic acid.

Additional exemplary farnesyl derivatives include, without limitation, FTS-methyl ester (FTS-ME), FTS-methoxymethylene ester (FTS-MOME), and FTS-amide (FTS-A) and 5-fluoro-FTS, as described hereinabove.

The anion of 2-((2E,6E)-3,7,11-trimethyldodeca-2,6,10-trienylthio)benzoic acid is also known as farnesylthiosalicylate (FTS) or salirasib. As demonstrated in the Examples section that follows below, a DES having FTS as a bioactive agent has been prepared and tested for its drug-eluting properties (drug-release profile), and was found highly effective as such.

In order to produce the composite structures described herein, and particularly such structures which combine properties such as desired morphologic and mechanical properties together with the capacity to contain bioactive agents while retaining their activity and to controllably release these agents, the present inventors have developed a unique process.

Thus, according to another aspect of the present invention there is provided a process of preparing the composite structures as described herein. The process is effected by providing a prefabricated core structure; providing an emulsion containing an aqueous solution and an organic solution which comprises a second polymer; contacting the core structure

and the emulsion to thereby obtain a core structure having a layer of the emulsion applied on at least a part of the core structure; and freeze-drying the core structure having the layer applied thereon.

The core structures constituting the composite structures of the present embodiments can be of natural or synthetic origins, and can be provided ready for use without further manipulation or preparation procedures or upon surface pre-treatment thereof.

For example, the meshes which can serve as the core structure of the composite structures of the present embodiments can be produced by conventional weaving or punching techniques

As mentioned hereinabove, in some embodiments of the present invention, the composite structures are biodegradable structures, comprising a biodegradable core and a biodegradable coat, each encapsulating one or more bioactive agents. In these cases, the core structure containing one or more bioactive agents can be prepared using any of the methods described in the art, while recognizing the compromised made with respect to the mechanical properties and physical dimension of the resulting structures.

As mentioned hereinabove, in addition to bioactive agent(s), additional ingredients, such as biodegradation promoting agents and other agents, can be added to the polymer in the process of preparing the core.

Once the structures which are used as a core for the composite structure of the present embodiments are produced or otherwise provided, the coat can be formed thereon by means of applying a layer of an emulsion onto the surface of the core structure. As mentioned hereinabove, the layer of the emulsion can cover parts of the core structure or the entire core structure, and further block the fine structural features of the core structure or be cleared therefrom. Discrete patches of the emulsion layer can be achieved by, for example, spraying, sputtering or brushing the emulsion on the surface of the core structure. Long continuous streaks (patches) of the emulsion along the core structure can be achieved, for example, by partially dipping the core structure in the emulsion without fully immersing the core structure in the emulsion; and a whole-surface sheath can be achieved by fully immersing the core structure in the emulsion.

The thickness of the coat depends on the viscosity of the emulsion, namely the more viscous the emulsion, the more it sticks to the core structure and thus the thicker the resulting coat is. Alternatively, the core structure can be dipped in the emulsion more than once so as to form a thicker layer of emulsion which turns into a thicker coat.

The term "emulsion" as used herein, describes a mixture of two immiscible liquids, typically referred to as phases, such as water and oil. One liquid (typically referred to as the dispersed phase) is dispersed in the other (typically referred to as the continuous phase).

The term "emulsifier" (also known as a surfactant or other surface active material) as used herein, refers to a substance which stabilizes an emulsion.

According to some embodiments, the emulsion used to form the porous coat of the composite structures presented herein is a "water-in-oil" or reversed (inverted) emulsion, wherein droplets of the aqueous phase are dispersed in the continuous organic phase.

The emulsion, according to some embodiments of the present invention, is provided by preparing two solutions, one being the aqueous phase (water-based phase) and another being the organic phase (oil-based phase).

The organic phase is prepared by dissolving one or more polymer in an organic solvent. The organic solvent is selected immiscible with an aqueous solution. Examples of such organic solvents include, without limitation, chloroform, dichloromethane, carbon tetrachloride, methylene chloride, xylene, benzene, toluene, hexane, cyclohexane, diethyl ether and carbon disulfide. Alternatively the organic solvent is chloroform, which is immiscible with water, and suitable for dissolving the abovementioned polymer, i.e., a biodegradable aliphatic co-polymer such as poly(DL-lactic-co-glycolic acid) at a ratio of DL-lactic acid to glycolic acid of about 75 weight percentage to about 25 weight percentage respectively. The content of the biodegradable polymer in the organic solvent may range, according to the present embodiments, from about 1 weight-to-volume percentage to about 50 weight-to-volume percentages, and from about 10 weight-to-volume percentages to about 25 weight-to-volume percentages.

The aqueous phase may contain solely water, or may contain additional substances, as detailed hereinbelow.

The bioactive agent can be introduced to either the organic or the aqueous phase, depending on its nature, namely a hydrophobic bioactive agent, which is miscible in the solvent of the organic phase is dissolved or otherwise introduced into the organic phase, while a hydrophilic/amphiphilic bioactive agent which is water-soluble, is introduced into the aqueous phase.

The presence of the bioactive agent in either one of the phases of the emulsion determines many factors of its release profile, as discussed hereinabove. A hydrophilic/amphiphilic agent which is dissolved in the aqueous phase will be found in the droplets of the dispersed phase and subsequently will be incorporated to the coat on the inner walls of the pores. A hydrophobic agent which is dissolved in the organic phase will be found in the continuous phase and subsequently will be incorporated to the solid material of coat surrounding the pores.

The organic or the aqueous phase may further include additional agents such as, for example, emulsifying agents (emulsifiers) which may be required to stabilize the emulsion, surfactants, anti-static agents, chelating agents, preservatives, solubilizers, viscosity modifying agents, biodegradation promoting agents, penetration enhancers and other additional agents as described hereinabove.

Buffer salts which are suitable for use in the preparation of the emulsion according to embodiments of the present invention include citrate buffers, acetic acid/sodium acetate buffers and phosphoric acid/sodium phosphate buffers.

Emulsifiers which are suitable for use in the preparation of the emulsion according to embodiments of the present invention include vegetable derivatives, for example, acacia, tragacanth, agar, pectin, carrageenan and lecithin; animal derivatives, for example, gelatin, lanolin and cholesterol; semi-synthetic agents, for example, methylcellulose and carboxymethylcellulose; and synthetic agents, for example, Carbopols®. Other emulsifiers include glycols and polyglycols, glycerides and polyglycerides, sorbates and polysorbates, sorbitan isostearate, sorbitan oleate, sorbitan sesquioleate, sorbitan trioleate, alkyl-amines and alkyl-amides, and esters, salts and mixtures thereof.

As used herein, the term "surfactant", which is also referred to herein interchangeably as "a surface-active agent" describes a substance that is capable of modifying the interfacial tension of the liquid in which it is dissolved.

As mentioned hereinabove, while preparing composite structures in which one or more bioactive agent(s) are contained within the coat, the above-described emulsion contains the bioactive agent(s). The bioactive agent(s) can be either in the organic phase and/or in the aqueous phase, depending on its solubility, stability and other characteristics, and the desired properties of the resulting structure.

Thus, for example, water-soluble bioactive agents such as proteins, peptides and growth factors are dissolved in the aqueous phase. In these cases, water-in-oil emulsions would result in polymeric coats in which the bioactive agent is encapsulated within the pores of the coat. As mentioned hereinabove, discrete pores are desired so as to affect prolonged release of the bioactive agent.

Water-insoluble bioactive agents such as, for example, cytotoxic drugs and anti-proliferative agents, are dissolved in the organic phase. In these cases, water-in-oil emulsions would result in polymeric coats in which the bioactive agent is encapsulated within the polymer composing the coat. As mentioned hereinabove, numerous and relatively small and interconnected pores are desired so as to affect efficient release of the bioactive agent via a maximized exposed surface area.

A combination of water-soluble bioactive agents that are encapsulated in the pores and water-insoluble bioactive agents that are encapsulated in the polymer composing the coat is also within the scope of the present invention.

When containing a bioactive agent, the aqueous phase is prepared at a temperature which would not harm the bioactive agent, or otherwise jeopardize its activity. Typically the temperature of the aqueous phase is kept under 37 °C. Similarly, other parameters of the preparation of the aqueous solution, such as pH, salinity and other chemical and physical parameters are kept at such levels as to preserve the activity of the bioactive agent(s).

The organic phase, when containing the bioactive agent, is prepared by selecting a solvent that would not affect the activity of the agent.

Once the two solutions, i.e., the organic solution/phase and the aqueous solution/phase are prepared or otherwise provided, the two solutions are mixed at a predetermined ratio to thereby obtain a mixture thereof.

As is demonstrated in the Examples section that follows, the ratio between the aqueous and the organic phase in the emulsion may affect the properties of the resulting structure, as well as the release profile of an encapsulated bioactive agent.

According to some embodiments of the present invention, the ratio of the aqueous solution and the organic solution in the mixture may range from about 1 parts of the organic solution to 1 part the aqueous solution to about 20 parts of the organic solution to 1 part the aqueous solution. The ratio of organic solution to aqueous solution depends on the specific requirements from the final product and its intended use.

Once the mixture is obtained, the process of emulsification is effected to thereby obtain the emulsion. The process of emulsification, which is well known to any artisan skill in the art, is effected by a mechanical stirrer, mixer or homogenizer until the desired consistency is achieved.

The rate (energy input) and the time of emulsification mixing determine the size of the resulting pores in the coat to a large extent. Rapid mixing for extended periods of time (typically using a homogenizer) will result in very fine pores in the porous coat. Such rapid mixing is typical in cases where the bioactive agent is dissolved in the organic phase and nano-sized pores are desired, as is detailed hereinabove.

As mentioned hereinabove, in cases where the bioactive agent has a limited solubility in water or is not soluble in water, the phase which will contain this bioactive agent is the organic (continuous) phase. In such cases, the aqueous phase may be used in order to introduce additional components such as buffers, emulsifying agents, surfactants, anti-static agents, chelating agents, solubilizers, viscosity modifying agents, biodegradation promoting agents and penetration enhancers. In these cases the pores formed by the water droplets in the polymer may be very small and will accelerate the biodegradation process by increasing the surface area of the biodegradable polymeric coat.

When containing a bioactive agent, the emulsification is effected at a temperature which would not harm the bioactive agent, or otherwise jeopardize its activity. Typically the emulsification is effected at temperature lower than 37 °C. Similarly, other mechanical parameters of the emulsification process are kept at such levels as to retain the activity of the bioactive agent(s).

As presented hereinabove, the resulting emulsion is applied onto the core structure so as to form a layer of the emulsion thereon. Once the mesh is fully or partially covered with the emulsion, the core structure is subjected to freeze-drying so as to solidify the emulsion and obtain the final composite structure of the present invention.

Alternatively, in cases where the fine structural features of the core structure, such as for example the openings of a mesh-based core structure, must stay free of any obstruction, the excess coating filling the openings can be removed prior to freezing the emulsion coating the core structure.

The phrase "freeze drying" (also known as lyophilization) as used herein is a dehydration process typically effected by deep-freezing the material, typically by flash-freezing in liquid nitrogen, and then reducing the surrounding pressure to allow the frozen solvent, typically water and organic solvents in the material to sublimate directly from the solid phase to gas, and solidify on a condenser or cold-trap.

If a freeze-dried substance is sealed to prevent the re-absorption of moisture, the substance may be stored at room temperature without refrigeration, and be protected against spoilage for extended periods of time. Freeze-drying tends to damage the tissue being dehydrated less than other dehydration methods, which involve higher temperatures.

According to some embodiments of the present invention, the process of freeze-drying, which is well known to any artisan skill in the art, is carried out at reduced temperature and pressure using conventional methods and tools. A porous coat is therefore the product of a freeze-dried water-in-oil emulsion wherein the droplets of the dispersed aqueous phase turn to voids or pores in the solidified continuous organic phase of the polymer. In cases where the aqueous phase contains at least one bioactive agent, the droplets of the dispersed aqueous phase become microscopic capsules containing the bioactive agent(s) which are encapsulated, entrapped and embedded in a solid polymer once the emulsion is freeze-dried.

Interim summing up, a wide range of bioactive agents can be incorporated into the coat of the composite structures described herein. The preparation of the coat does not involve harsh conditions which typically abolish the activity of many bioactive agents. The preparation of the coat via the formation of an emulsion comprising an aqueous phase and an organic phase enables the incorporation of bioactive agents having a hydrophilic/amphiphilic nature or a hydrophobic nature, and of a small organic molecule or a complex macro-biomolecule.

The incorporation of a bioactive agent having a more pronounced solubility trait, such as a small and predominantly hydrophobic drug molecule, requires a different treatment in order to be incorporated successfully in a composite structure as presented herein. A hydrophobic drug molecule is intuitively added to the organic phase where it is more soluble, and the use of surfactants may be required in order to stabilize the emulsion.

As discussed hereinabove, the composite structure of the present invention is designed suitable for use as medical devices and/or drug delivery systems in many medical procedures.

Hence, according to a further aspect of the present invention there is provided a medical device which is based on the composite structure described herein, which include, without limitations, stents, wound dressings, skin patches, suture anchors, interference and a general screws, angioplastic plugs, pins or rods, tacks, plates, anastomosis clips or rings, dental implants and guided tissue matrices.

In some embodiments of the present invention, the medical device is a biodegradable device.

Generally, the main motivation to have a biodegradable medical device is to have a device that can be used as an implant and will not require a second surgical intervention for removal. In its simplest form, a biodegradable device having a bioactive agent delivery capacity consists of a dispersion of the bioactive agent in a polymeric coat matrix. The bioactive agent is typically released as the biodegradable polymeric coat biodegrades *in vivo* into soluble products that can be absorbed and/or metabolized and eventually excreted from the body over a period of time which depends on the polymer and the physical dimensions of the device.

The term "delivering" or "delivery" as used in the context of the present embodiments refers to the act of enabling the transport of a substance to a specific location, and more specifically, to a desired bodily target, whereby the target can be, for example, an organ, a tissue, a cell, and a cellular compartment such as the nucleus, the mitochondria, the cytoplasm, etc.

According to a particular embodiment, the medical device based on the composite structure described herein is used for implantation, injection, or otherwise placed totally or partially within the body.

Exemplary devices which can be used for implantation application include, without limitation, a drug-eluting stent.

According to other embodiments of the present invention, the medical device is adapted for transdermal and/or topical applications in a subject. It is particularly important that such medical device would cause minimal tissue irritation when used to treat a given tissue.

Exemplary devices which can be used for transdermal application include, without limitation, a suture mesh, an adhesive plaster and a skin patch.

Exemplary devices which can be used for topical application include, without limitation, a suture mesh, an adhesive strip, a bandage, an adhesive plaster, a wound dressing and a skin patch.

According to other embodiments, the medical device is adapted for implanting the medical device in a bodily organ of a subject.

Exemplary devices are delineated hereinabove.

Examples of bodily sites where a medical device may be used include, without limitation, skin, scalp, a dermal layer, an eye, an ear, a small intestines tissue, a large intestines tissue, a kidney, a pancreas, a liver, a digestive tract tissue or cavity, a respiratory tract tissue or cavity, a bone, a joint, a bone marrow tissue, a brain tissue or cavity, a mucosal membrane, a nasal membrane, the blood system, a blood vessel, a muscle, a pulmonary tissue or cavity, an abdominal tissue or cavity, an artery, a vein, a capillary, a heart, a heart cavity, a male reproductive organ, a female reproductive organ and a visceral organ.

Medical devices, according to some mebodiments, include stents, wound dressings, sutures meshes and suture anchors, interference and general screws, angioplastic plugs, pins and rods, tacks, plates, strips, anastomosis clips and rings, dental implants, guided tissue matrixes and other medical devices as presented hereinabove.

According to other embodiments of the present invention the composite structures described herein can be used in the manufacturing of a wide variety of articles, which include, without limitation, fishing nets, insect and bird nets, vegetation nets, woven and non-woven cloths, disposable women's sanitary items, disposable facial masks (as used by surgeons), wet "paper" tissues (wipes), disposable underwear, disposable handkerchiefs, towels and diapers, disposable medical supplies, disposable food containers or dishes, disposable items of clothing, disposable cutlery items and other disposable consumer and industrial products.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Additional objects, advantages, and novel features of the present invention will become apparent to one ordinarily skilled in the art upon examination of the following examples, which are not intended to be limiting. Additionally, various embodiments and aspects of the present invention as delineated hereinabove and as claimed in the claims section below finds experimental support in the following examples.

## EXAMPLES

Reference is now made to the following examples, which together with the above descriptions, illustrate some embodiments of the invention in a non limiting fashion.

### *EXAMPLE 1*

### *DRUG ELUTING COMPOSITE MESHES*

***Mesh-based composite structures -A general introduction:***

It is demonstrated hereinbelow that a plainly-woven fabric woven from biodegradable polyglyconate fibers, coated and bound together, provides a continuous porous matrix which can give a wound dressing made therefrom an occlusive nature. The reinforcing fibers' excellent mechanical properties afford good mechanical strength whereas the continuous binding matrix can be tailored to afford desired properties, such as, drug release kinetics, water absorbance and other physical properties that promote wound healing.

In practice, a wound dressing can be woven from a combination of several types of fibers to create a release profile superimposed of several release profiles or drug types.

***Materials and experimental methods:***

MAXON™ bioresorbable polyglyconate monofilament surgical suture fibers (0.20-0.25 mm in diameter), by United States Surgical Inc., USA, were used as core structures.

Bioresorbable porous structures (the shell coating) were made of 50/50 poly(DL-lactic-co-glycolic acid) (PDLGA), inherent viscosity (i.v.) = 0.56 dL/g (in $CHCl_3$ at 30 °C, MW approximately 100 KDa), Absorbable Polymer Technologies, Inc., USA.

Poly(DL-lactic-co-glycolic acid), 50/50 % (50/50 PDLGA, inherent viscosity = 0.56 dL per gram in $CHCl_3$ at 30 °C, MW approximately 100 KDa), and 75/25 %, (75/25 PDLGA, cat. 75DG065, inherent viscosity = 0.65 dL per gram in $CHCl_3$ measured at 30 °C, molecular weight of approximately 97.1 KDa), obtained from Absorbable Polymer Technologies, Inc, USA, was used to form a biodegradable porous coat.

Gentamicin sulfate (cell-culture tested), 590 mg gentamicin base per mg, was obtained from Sigma-Aldrich

(cat. G-1264).

4-Aminomethylbenzenesulfonamide acetate salt (Mafenide acetate), was obtained from Sigma-Aldrich (cat. A-3305).

Ceftazidime hydrate, 90-105 %, was obtained from Sigma-Aldrich (cat. C-3809).

Bovine Serum Albumin (BSA), molecular weight = 66,000 Da, was obtained from Sigma-Aldrich (cat. A-4503).

Poly(vinyl alcohol) (PVA), 87-89 % hydrolyzed, molecular weight = 13,000-23,000 Da, was obtained from Sigma-Aldrich (cat. 36,317-0).

The polysorbate Span 80 (Sorbitan monooleate), molecular weight 428.608 g/mol, was obtained from Sigma (cat. no. 85548).

Isopropyl alcohol (propanol) was purchased from Frutarom, Israel.

O-phthaldialdehyde (P0657), sodium borate 0.04 M (B0127) and 2-hydroxyethylmercaptan (63690) were obtained from Sigma-Aldrich.

1,1,1,3,3,3-Hexafluoro-2-propanol (H1008) was purchased from Spectrum Chemical Mfg. Corp.

***Core structure surface treatment and mesh preparation:***

The core mesh structures were prepared (weaved manually) from MAXON™ (polyglyconate) surgical suture fibers, which were surface-treated prior to the weaving process in order to dispose of the original coating and thus enhance the adhesion between the core elements and the coating layer. The fibers were placed in special holders and dipped in 1,1,1,3,3,3 hexafluoro-2-propanol for 40 seconds. Thereafter the fibers were washed with 70 % ethanol and air dried. Mesh structures were prepared by cross weaving the surface-treated fibers.

Figure 2A presents a schematic illustration of a pre-fabricated mesh structure as can be prepared from MAXON™ (polyglyconate) surgical suture fibers.

***Preparation of the emulsion for the coating layer:***

As an exemplary general procedure, a pre-measured amount of PDLGA is dissolved in chloroform to form an organic solution. A pre-measured amount of a drug is dissolved in double-distilled water and then poured into the organic phase in a test tube. Homogenization of the emulsion is performed using a Kinematica PT-3100 Polytron homogenizer operating at 16,000 -18,000 rpm (medium rate which was found to be optimal) for 2 minutes. The emulsion formulation for the composite mesh samples contains, for example, 15 % w/v polymer in the organic solution, 5 % w/w drug in the aqueous medium (relative to the polymer content), 1 % albumin in the aqueous medium, and an organic to aqueous (O:A) phase ratio of 6:1 v/v.

The following exemplary formulations were prepared according to the general procedure hereinabove:

Formulation ALB1, prepared with O:A= 6:1 containing 1 % w/v (relative to the aqueous volume) BSA as a surfactant;
Formulation ALB2, prepared with O:A= 12:1 containing 1 % w/v (relative to the aqueous volume) BSA as a surfactant; and
Formulation SPA1, prepared with O:A= 12:1 containing 1 % w/v (relative to the organic phase volume) Span 80 as a surfactant.

***Preparation of core/coat composite meshes:***

Wound dressings loaded with antibacterial drugs can be prepared either by using the composite drug-loaded fiber for a basic element, as taught in U.S. Patent Application having Publication No. 20070134305, or by preparing a mesh-like structure based on the core fibers, dip-coating the mesh in an inverted emulsion containing the drug molecules and then freeze drying the coated structure. According to some embodiments of the present invention, the method for preparing drug eluting mesh-based wound dressings, comprises coating an entire structure.

Exemplary drug-eluting meshes were prepared using a series of antibacterial drugs such as gentamicin, ceftazidime and mafenide acetate. A pre-fabricated mesh, woven from MAXON™ suture fibers, was dipped in the inverted emulsion and then freeze dried. Figure 2B presents a schematic illustration of an antibiotic-eluting mesh structure, coated with a layer which was formed by freeze-drying a coat of drug-loaded emulsion.

The woven suture fiber structure was then dip-coated while placed on holders in fresh emulsions and immediately thereafter flash-frozen in a liquid nitrogen bath. The samples were then placed in a pre-cooled (-105 °C) freeze-dryer (Virtis 101 equipped with a nitrogen trap) in order to preserve the microstructure of the emulsion-based structures. Drying was performed in two stages:

The freeze-dryer chamber pressure was reduced to 100 mTorr while the temperature remained at -100 °C. After 3

hours a hot plate was turned on to -45 °C for an additional 12 hours. Thereafter the condenser was turned off and its plate temperature gradually increased to room temperature while the pressure was monitored between 13332 Pa and 93326 Pa (between 100 mTorr and 700 mTorr). During this step the liquid nitrogen trap condensed the excess water and solvent vapors. The samples were stored in desiccators until use.

***Morphological characterization:***

The morphology of the wound dressing's structures was observed using a Jeol JSM-6300 scanning electron microscope (SEM) at an accelerating voltage of 5 kV. Surfaces of cryogenically fractured surfaces were sputtered with paladium prior to observation. The mean pore diameter (n=100 pores) and porosity of the observed morphologies was analyzed using Sigma Scan Pro software and statistics were calculated using SPSS 10 software. Statistical significance was determined using the ANOVA (Tukey-Kramer) method. The area occupied by the pores was calculated for each SEM fractograph using the Sigma Scan Pro software in order to evaluate the porosity of the samples. The porosity was determined as the area occupied by the pores divided by the total area.

***Water vapor transmission rate:***

The moisture permeability of the wound dressing was determined by measuring the water vapor transmission rate (WVTR) across the composite core/coat structure. A Sheen Payne permeability cup with an exposure area of 10 $cm^2$ was filled with 10 ml of PBS and mounted with a circular wound dressing. The cup was placed in a straight position inside an oven at 37 °C, containing 1 Kg of freshly dried silica gel in order to maintain relatively low humidity conditions. The weight of the assembly was measured every hour for 12 hours and a graph of the water evaporated versus time was plotted. WVTR was calculated by the formula: $WVTR = \frac{slope \times 24}{area}\left[\frac{g}{m^2 \cdot day}\right]$.

***Water uptake aptitude:***

Fluid absorbing capacity of any wound dressing is an important criterion for maintaining a moist environment over the wound bed. Water uptake of the composite core/coat structure wound dressing was measured over a 7 days. Dry wound dressings were cut into 1cm X 1.5cm rectangles, weighed and placed in bottles containing 2 ml PBS (pH 7.0). The bottles were closed and placed in an incubator at 37 °C. The weight of samples was measured after 6 hr, 12 hr, 1, 2, 3 and 7 days by removing the PBS and blotting them gently to remove excess fluid. The water uptake was calculated as $\frac{W_{wet} - W_{dry}}{W_{dry}} \times 100$.

***Tensile mechanical properties:***

The wound dressing's tensile mechanical properties were measured at room temperature, under unidirectional tension at a rate of 10 mm/min, using a 5500 Instron machine. The wound dressing was cut into a dog bone shape (neck length 5 cm, width 1 cm). The tensile strength was defined as the maximum strength in the stress-strain curve. The maximal strain was defined as the breaking strain. Young's modulus was defined as the slope of the stress-strain curve in the elastic (linear) region. Four samples were tested for each type of specimen. Other specimens were immersed in phosphate buffered saline (PBS), pH 7.0, at 37 °C for 1, 2 and 3 weeks, after which they were dried and tested in the same manner.

The means and standard deviations were calculated using the SPSS 10 software. ANOVA (Tukey-Kramer) was used for group comparison.

***In-vitro drug release studies:***

The composite core/coat fiber and mesh structures were immersed in phosphate buffered saline (PBS) at 37 °C for 60 days in order to determine the various drug release kinetics from these structures. The release studies were conducted in closed glass vessels containing 1.5 ml PBS medium. The medium was completely removed periodically, at each sampling time (6 hours, 1, 2, 3, 7, 14, 21, 28, 35, 42, 49, and 56 days), and fresh medium was introduced. The experiments were performed in triplicate.

***Gentamicin assay:***

Determination of the medium content of gentamicin was performed by using an Abbott Therapeutic Drug Monitoring System- TDX (Abbott Laboratories) according to the directions of the manufacturer. This completely self-contained machine enables to determine the concentration of gentamicin based on a polarization fluoroimmun-assay, using fluorescein as a tracer. Briefly, fluorescein is excited by polarized light, and the polarization of the emitted light depends on the molecular size. Labeled and unlabeled drug molecules thus compete for binding sites. The concentration of drug in the sample is proportional to the scatter of polarized light caused by free/labeled drug molecules. The measurable concentration range without dilution is 0 to 10 μg/ml. Higher concentrations were measured following manual dilution.

***Mafenide acetate assay:***

The mafenide content of the medium samples was determined using Jasco High Performance Liquid Chromatography (HPLC) with a UV2075 plus detector and a reverse phase column (Interstil® ODS-3V 5 μm, inner diameter 4.6 mm, length 250 mm), kept at 25 °C. The mobile phase consisted of a mixture of PBS and acetonitrile (100/0, v/v) at a flow rate of 1 ml per minute with a quaternary gradient pump (PU 2089 plus), gradient t=0 minutes, 100/0, t=1.5 minutes, 90/0, t=4 minutes, 100/0. Samples of 30 μl were injected with an autosampler (AS 2057 Plus). The column effluent was eluted for 9 minutes and detected at 267 nm. The area of each eluted peak was integrated using the EZstart software version 3.1.7. A calibration curve was prepared for the range of concentrations 1.0 to 200.0 μg/ml (correlation coefficient >0.999, slope: 0.0002295).

***Ceftazidime assay:***

The ceftazidime content of the medium samples was determined using Jasco High Performance Liquid Chromatography (HPLC) with aUV2075 plus detector and a reverse phase column (INTERSTIL® ODS-3V 5 μm, inner diameter 4.6 mm, length 250 mm), kept at 25 °C. The mobile phase consisted of a mixture of PBS and acetonitrile (95/5, v/v) at a flow rate of 1 ml per minute with a quaternary gradient pump (PU 2089 plus) without gradient. Samples of 20 μl were injected with an autosampler (AS 2057 Plus). The column effluent was eluted for 22 minutes and detected at 254 nm. The area of each eluted peak was integrated using the EZstart software version 3.1.7. A calibration curve was prepared for the range of concentrations 1.0 to 200.0 μg/ml (correlation coefficient >0.999, slope: 0.0000318).

***Residual drug recovery from composite fibers:***

Residual drug recovery from the composite fibers and meshes was measured by placing the samples in 1 ml methylene chloride in order to dissolve the remaining PDLGA coating. Thereafter, water (2 ml) was added in order to dissolve the hydrophilic drug residues. The materials were vortexed for 30 seconds and then were left to stand until phase separation occurred. The aqueous phase was then filtered in order to dispose of polymer particles and the drug concentration was determined using one of the assays described hereinabove.

The encapsulation efficiency was determined as the actual drug concentration encapsulated in the composite structures divided by the theoretical value (the quantity that was added to the emulsion when it was created).

A similar process of drug recovery (from fresh structures not used for the drug release study) was used in order to evaluate the encapsulation efficiency and to elucidate the effect of the emulsion formulation on the encapsulation efficiency. The experiments were performed in triplicate.

***Results:***

***Morphological characterization:***

A composite wound-dressing composed of plain-woven mesh of polyglyconate fibers, bound by a continuous poly-(DL-lactic-co-glycolic acid) (PDLGA) porous matrix, loaded with the antibiotic ceftazidime, was produced and studied as presented hereinabove.

Figures 3A-D present SEM fractographs of an exemplary plain-weave composite structure useful as basic wound dressing according to some embodiments of the present invention, showing a basic unit of the composite structure (Figure 3A wherein the white bar represents 1 mm), a cross-section of the PDLGA coat matrix which is well adhered to the core suture fibers, forming a coat layer connecting the core fibers (Figure 3B), and a magnified view of the coat's cross-section having a thickness of about 60 μm (Figure 3C wherein the white bar represents 50 μm and Figure 3D wherein the white bar represents 5 μm).

As can clearly be seen in Figures 3A-D, employing the freeze-drying of inverted emulsion technique to create

the PDLGA binding matrix, resulted in a porous microstructure which also acts as a reservoir for bioactive agents, such as antibiotics, incorporated therein.

Figures 4C-A present SEM fractographs, showing the effect of change in inverted emulsion formulation parameters on the microstructure of the resulting freeze-dried coating matrix containing 5 % w/w ceftazidime and 15 % w/v polymer (50/50 PDLGA, MW 100 KDa), and O:A phase ratio of 6:1, 1 % w/v BSA (Figure 4A), O:A phase ratio of 12:1, 1 % w/v BSA (Figure 4B), and O:A phase ratio of 12:1, 1 % w/v Span 80 (Figure 4C).

As can be seen in Figures 4A-C, the resulting microstructures of the matrix attained for these three different emulsion formulations were different, and the numerical results are presented in Table 1 below.

***Table 1***

| Emulsion Formulati on | O:A ratio | Drug content * (w/w) | Polymer content in the organic phase * (w/w) | Surfactant content ** | Microstructure | |
|---|---|---|---|---|---|---|
| | | | | | % Porosity | Pore Diameter μm |
| BSA1 | 6:1 | | | BSA (1 % w/v in the aqueous phase) | 63±4 | 1.4±0.3 |
| BSA2 | 12:1 | 5% | 15% | BSA (1 % w/v in the aqueous phase) | 35±2 | 1.4±0.3 |
| SPA1 | 12:1 | | | Span 80 % (1 % w/v in the organic phase) | 56±3 | N/A |

* Relative to the polymer weight

** Relative to a liquid phase volume (organic or aqueous)

As can be seen in Table 1, the microstructure of the reference formulation BSA1 (Figure 4A), is highly porous with an average porosity of 63±4 % and pore diameter of 1.4±0.3 μm. Increasing the emulsions' organic:aqueous (O:A) phase ratio from 6:1 to 12:1 (formulation BSA2), resulted in larger polymer domains in between pores, less pore connectivity, and lower porosity 35±2 % (Figure 4B), however, it did not affect the overall pore size significantly, which remained 1.4±0.3 μm.

***Water vapor transmission rate:***

Water Vapor Transmission Rate (WVTR) was measured for the three aforementioned types of composite wound dressing structures, based on the formulations presented in Table 1.

Figures 5A-B present comparative plots, showing water mass loss as a function of time (Figure 5A), wherein the results measured from uncovered surface are marked with solid black rectangles, results measured from composite structures made with emulsion formulation ALB1 are marked with solid blue circles, results measured from composite structures made with emulsion formulation ALB2 are marked with solid red rectangles, results measured from composite structures made with emulsion formulation SPA1 are marked with solid green triangles and results measured from dense PDLGA (50/50, MW 100 KDa) film are marked with white rectangle, and showing the water vapor transmission rates (WVTR) for the various wound dressings (Figure 5B).

As can be seen in Figures 5A-B, evaporative water loss through the various composite structures was linearly dependant on time ($R^2 > 0.99$ in all cases), with constant WVTR as a result. A WVTR of 3452±116 grams per square meter per day was measured for a composite structure dressing based on the emulsion formulation BSA1. When the O:A phase ratio was increased to 12:1 (emulsion formulation BSA2), the WVTR was reduced significantly to 480±69 grams per square meter per day. When the surfactant BSA was replaced with Span 80 (emulsion formulation SPA1), a WVTR of 2641±42 grams per square meter per day was recorded. WVTR was determined experimentally also for a dense PDLGA film, to serve an analogue to currently available biodegradable films currently used in wound care. A WVTR of 356±106 grams per square meter per day was recorded in this case. In addition, the WVTR of an exposed aqueous surface, 6329±725 grams per square meter per day, was determined experimentally to simulate a situation in which no composite structure dressing is applied on the wound surface.

***Water uptake:***

Two exemplary composite structures, formed in the shape of wound dressing sample with distinctly different porosities were studied: (i) a dressing derived from the reference emulsion formulation BSA1, and (ii) a dressing in which

the O:A phase ratio was modified from 6:1 to 12:1 (emulsion formulation BSA2). Dressings were placed in PBS (pH 7.0) to simulate the water absorption behavior in the presence of wound fluids and water absorption ability was calculated according to the formula described in the experimental section. Both types of dressings displayed similar temporal adsorption patterns, consisting of a quick initial uptake within the first 24 hours, followed by a slight decrease in water content at three days and then by a steady increase over the two following weeks.

Figures 6A-B present comparative plots, showing the water uptake as a function of time as measured for exemplary composite wound dressing structures coated with two different emulsion formulations, wherein the results measured from the structure coated with an emulsion containing 5 % w/w ceftazidime, 15 % w/v polymer (50/50 PDLGA, MW 100 KDa), O:A phase ratio of 6:1, stabilized with 1 % w/v BSA are marked with solid blue diamonds, and the results measured from the structure coated with a similar emulsion formulation having an O:A modified to 12:1 are marked with solid red rectangles.

As can be seen in Figures 6A-B, a considerable share of the initial water uptake occurred in the first 6 hours (Figure 6A). Dressings of type (i) increased 65 % in weight at this stage whereas dressings of type (ii) increased by 56 % w/w. At three days, water uptake in both types of dressing decreased to approximately 45 % w/w and thereafter steadily increased till reaching a 125 % increase in weight after three weeks.

***Tensile mechanical properties:***

The effect of polymer degradation on the mechanical properties of the composite wound dressing structures, according to some embodiments of the present invention, was determined for the dressing based on the reference emulsion formulation BSA1.

Figures 7A-D present the results of the mechanical properties studies conducted for composite wound dressing structures, showing the tensile stress-strain curves for wound dressings immersed in water for 0 weeks (purple line in Figure 7A), 1 week (red line in Figure 7A), 2 weeks (green line in Figure 7A), and 3 weeks (blue line in Figure 7A), comparing the elastic modulus measured for these samples (Figure 7B), and the tensile strength (Figure 7C) and maximal tensile strain (Figure 7D), as a function of immersion time evaluated from the tensile stress-strain curves, whereas the comparisons were made using analyses of variance and significant differences (marked with *).

As can be seen in Figures 7A-D, the composite wound dressing structures, incubated for the durations of 1, 2 and 3 weeks, displayed similar tensile strengths of about 21-27 MPa, and strains at break of 55-63 %. When breakage had occurred, it was initiated due to failure of the reinforcing fibers and not by the matrix. The initial Young's modulus of the dressing (126$\pm$27 MPa) was preserved after one week incubation (117$\pm$19 MPa), however then decreased after two weeks (72$\pm$11 MPa), and remained unchanged after three weeks (70$\pm$26 MPa).

***In vitro drug-release studies:***

The results of the drug-elution assay are summarized in Figure 8, which presents comparative plots of eluted drug concentrations as a function of time as measured from various drug-eluting mesh-based composite structures prepared according to embodiments of the present invention, wherein the results obtain for gentamicin sulfate-eluting meshes are marked by circles, ceftazidime pentahydrate-eluting meshes are marked by triangles and the results obtain for mafenide acetate-eluting meshes are marked by diamonds, and whereas the formulation parameters of the emulsion used to form the coat of the meshes are: 15 % (w/v) polymer in the organic phase, 5 % (w/w) drug concentration, phased ratio of organic to aqueous 6:1 and 1 % albumin as a surfactant.

All three exemplary antibiotic drugs featured resemblance in form, and thus the pore-size resulting from the emulsions converged to 1-1.3 $\mu$m in diameter, suggesting that albumin is an effective surfactant for the stabilization of such drugs.

Albumin may also act as a binding agent for various drugs through specific and non-specific interactions, as it is well-known for albumin. Attempts to employ this principle in albumin sealed vascular grafts soaked in antibiotics have already been reported [14; 15].

Sulfonamides (mafenide) have been found to bind to serum proteins and in particular to albumin from 20 % to more than 90 %, more than ceftazidime and gentamicin. The extent of binding depends on the agents' pKa, and in general, the lower the pKa, the greater the binding. This is the most reasonable explanation why mesh-based composite structures containing mafenide in combination with albumin display a significantly lower burst release and a moderate release rate compared to analogous formulations containing the other two antibiotics, especially as the microstructural differences between the three drugs are reduced when albumin is used and therefore their contribution can be ruled out.

*EXAMPLE 2*

***DRUG-ELUTING COMPOSITE STENTS***

***General concept:***

The viability of eluting a variety of small-molecule drugs, having different chemical properties and biological activities, from a porous layer of a degradable material, prepared according to some embodiments of the present invention, was demonstrat5ed. Both, fibers and more complex structures coated with the drug-loaded porous layer, were used. For demonstration purposes, a simple fiber was used to study the elution and drug-releasing parameters by the currently presented system. Since most complex structures can be modeled by a set of fibers, the results presented for fibers are considered applicable for these complex structures, such as stents and meshes.

***Materials and experimental methods:***

Maxon™ polyglyconate monofilament (3-0) suture fibers, with a diameter of 0.20-0.25 mm (Syneture, USA), containing a 67.5:22.5 glycolide to trimethylene carbonate ratio, were used as core fibers to model more complex structures. These suture fibers were surface-treated in order to enhance the adhesion between the fiber and the coating. Hence, the polyglyconate fibers were slightly stretched on special holders and dipped in 1,1,1,3,3,3-hexafluoro-2-propanol (hexafluorisopropanol) for 40 seconds, and then washed with ethanol and dried at room temperature.

Bioresorbable porous coating layers were made of 75/25 poly(DL-lactic-co-glycolic acid), referred to herein and throughout as 75/25 PDLGA, is characterized by inherent viscosity of 0.65 dL/g in $CHCl_3$ at 30 °C, approximately 97,100 g/mole); and 50/50 poly(DL-lactic-co-glycolic acid), referred to herein and throughout as 50/50 PDLGA, is characterized by inherent viscosity of 0.56 dL/g in $CHCl_3$ at 30 °C, approximately 31,300 g/mole); were both obtained from Absorbable Polymer Technologies Inc., USA.

Farnesylthiosalicylate (FTS, Salirasib, see structure below) was received from Concordia Pharmaceuticals (Sunrise, FL, USA).

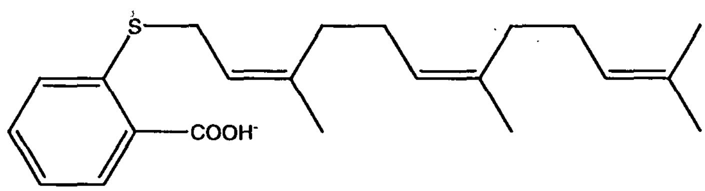

Farnesylthiosalicylate (FTS, Salirasib)

The incorporation of this relatively new hydrophobic drug, FTS, in a stent coating is expected to overcome the incomplete healing and lack of endothelial coverage associated with at least some of the current drug eluting stents.

Paclitaxel (Genexol™), a relatively hydrophobic molecule, was purchased from Sam Yang Corp, Seoul, Korea.

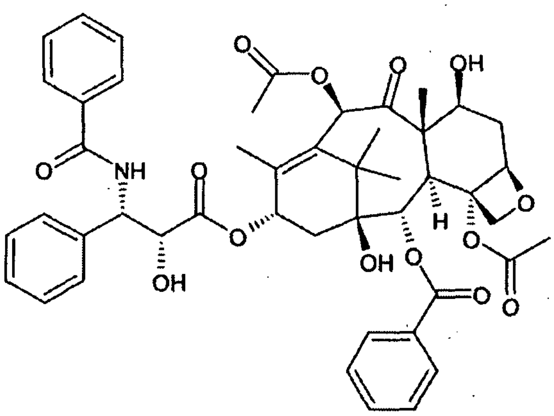

Paclitaxel

Bare stainless steel platform stents, having a balloon diameter of 5.0 mm, and a length of 13 mm (a catheter/stent assembly, lot 08052857, ref. 352370), were obtained from Pro-Kinetic, Biotronik Switzerland.
Chloroform, $CHCl_3$, HPLC grade and methylene chloride, $CH_2Cl_2$, HPLC grade were purchased from Frutarom, Israel.
Acetonitrile, $CH_3CN$, HPLC grade was purchased from J.T. Baker.

***FTS chemical stability:***

Two milligrams of FTS were placed in 3 ml PBS at 37 °C for 100 days in order to determine its stability throughout the release experiment. The closed scintillation vials containing the FTS in PBS were placed in a horizontal bath shaker operated at a constant rate of 130 rpm. The FTS content of a single vial was measured at each point of time.
FTS was extracted as follows: 3 ml methylene chloride were added to the 3 ml PBS/FTS mixture and stirred for 10 minutes in order to dissolve the drug. One milliliter was carefully removed from the bottom of the vial (the organic phase) using a pipette and placed in a new vial. Five milliliters of 50/50 acetonitrile/double-distilled water (the medium) were added to the vial and then evaporated under nitrogen conditions. The content of each vial was transferred to a test tube and diluted to 20 ml. The FTS content of the medium samples was determined using HPLC, as described here.

***Preparation of the emulsion for the coating layer:***

A pre-measured amount of 50/50 PDLGA or 75/25 PDLGA was dissolved in chloroform to form an organic solution and FTS or paclitaxel was added to the solution. Doubly-distilled water was then poured into the organic phase (in a scintillation vial) and homogenization of the emulsion was performed using a homogenizer (Polytron PT3100 Kinematica, 12 mm rotor) operating at 16,500 rpm (medium rate) for 2 minutes, for most investigated samples. An emulsion formulation containing 12.5 % w/v polymer in the organic solution, chloroform, 2% w/w FTS (relative to the polymer content), and an organic to aqueous (O:A) phase ratio of 4:1 v/v was used as a reference sample for the FTS formulation. Other formulations included 20 % w/v polymer, 1 % and 4 % w/w FTS, O:A phase ratios of 2:1 and 8:1 and copolymer composition of 75/25 PDLGA.
Some samples were prepared using homogenization rates of 8,500 rpm (low rate) or 22,500 rpm (high rate) in order to study the effect of processing kinetics on the porous shell structure.
The emulsion formulation selected for the stent coating contained 7.2 % w/v polymer in the organic solution, 3.4 % w/w FTS (relative to the polymer load), and an organic to aqueous (O:A) phase ratio of 4:1 v/v.

***Fiber and Stent coating:***

The treated core polyglyconate fibers were dip-coated (while placed on holders) in fresh emulsions and then frozen immediately in a liquid nitrogen bath. The holders and the samples were then placed in a pre-cooled (-105 °C) freeze dryer (Virtis 101 equipped with a nitrogen trap) capable of working with organic solvents (freezing temperature of the condenser was approximately -105°C) and freeze dried in order to preserve the temporal state of the emulsion

in a solid form. The freeze dryer chamber pressure was reduced to 100 mTorr while the temperature remained constant (-105°C) in order to sublimate the water and solvents. Room temperature was then slowly restored in order to evaporate residual solvent vapors. The samples were then stored in desiccators until use. At the end of the process, the shell's microstructure reflects the emulsion's stability.

The catheter/stent assembly was connected to a manual pump (Guidant cooperation) and the balloon was inflated to a pressure of 14 Bar, which corresponds to an inner diameter of 5.27 mm). The stent was detached from the catheter in a completely open state, dipped in fresh emulsions using tweezers, and then air pressure (6 Bar) was applied in order to remove excess emulsion between the struts. The coated stents were dipped immediately in a liquid nitrogen bath, and then placed in a pre-cooled (-105 °C) freeze dryer (Virtis 101 equipped with a nitrogen trap) capable of sublimating (drying) organic solvents (freezing temperature of the condenser was approximately -105 °C) and allowed to freeze-dry in order to preserve the microstructure of the emulsion in a rigid form. The freeze-dryer chamber pressure was reduced to 100 mTorr while the temperature remained in a constant temperature of -105 °C in order to sublimate the water and organic solvents. The chamber was slowly warmed to room temperature in order to evaporate residual solvents vapors, and the finished samples were then stored in desiccators.

***Morphological characterization:***

The morphology of the drug-eluting stents' coating was visualized using a Jeol JSM-6300 scanning electron microscope (SEM) and an accelerating voltage of 5 kV. The stents were sputtered with gold prior to observation using standard techniques. The morphology of other composite core/coat structures (cryogenically fractured surfaces) was observed in the same manner.

All quantitative measurements were summarized as means±standard deviations, unless otherwise specified. Comparisons of the mean pore diameter were carried out for the microstructure analysis using the unpaired student's *t*-test for two group comparisons, or ANOVA (post-hoc Tukey-Kramer) for three group comparisons. SPSS was used for all statistical calculations. Statistical significance was determined at $p<0.05$.

*In vitro* morphology of wet shell structures was characterized using an environmental SEM (Quanta 200 FEG ESEM) using an accelerating voltage of 10 KV in a pressure of 600 Pa (4.5 Torr). Fractographs were taken at three time points while the specimens were maintained in double-distilled water outside the ESEM. The specimens were fixed to a special base, and initial coordinates were recorded so as to enable good return to these coordinates.

***In vitro drug release studies:***

The composite drug-eluting fibers were immersed in phosphate buffered saline (PBS) at 37 °C and pH 7.4 for 35 days (FTS) or 37 weeks (paclitaxel), in triplicates, in order to determine the release kinetics from the drug-loaded composite structures. Each test vial contained 2 fibers; each fiber was 5 cm long. The release studies were conducted in closed glass tubes containing 3 ml PBS medium, using a horizontal bath shaker operated at a constant rate of 130 rpm. The medium was removed completely at certain sampling time points, extracted from the aqueous medium as described herein and measured using HPLC. Fresh medium was then introduced. At the end of the experiment the fibers were immersed in methylene chloride and the residual amount of drug was measured.

The drug content of the medium samples was determined using Jasco High Performance Liquid Chromatography (HPLC) equipped with a UV 2075 plus detector and a quaternary gradient pump (PU 2089 plus). A reverse phase column (ACE 5 C18, inner diameter 4.6 mm, length 250 mm) was used for FTS measurements, equipped with a column guard and kept at room temperature (25 °C). The mobile phase consisted of a mixture of acetonitrile and phosphate buffer (30 mM, pH 4.5) at a ratio of 70/30 v/v, respectively, at a flow rate of 1 ml/min without gradient. The paclitaxel content of the medium samples was determined using a reverse phase column (Zorbax ODS 5 μm, inner diameter 4.6 mm, length 150 mm), and kept at 25 °C. The mobile phase consisted of a mixture of acetonitrile and double-distilled water (55/45, v/v) at a flow rate of 1 ml/min. 100 μl samples were injected with an autosampler (AS 2057 Plus). UV detection was carried out at 227 nm for paclitaxel and 322 nm for FTS. The area of each eluted peak was integrated using the EZstart software version 3.1.7.

Two FTS-eluting stents were immersed in phosphate buffered saline (PBS) at 37 °C and pH of 7.4 for 28 days in order to determine the release kinetics of FTS. The drug-release profile studies were conducted in closed scintillation vials containing 3 ml PBS medium, using a horizontal bath shaker operated at a constant rate of 130 rpm. The medium was removed completely periodically at certain sampling time intervals, and fresh medium was introduced. At the end of the trial the stents were immersed in methylene chloride and the residual drug amount was measured.

***FTS extraction procedure:***

FTS or paclitaxel extraction from the medium was performed as follows: 3 ml PBS/drug medium was completely

removed at each time point and placed in a scintillation vial. 3 ml acetonitrile and 1 ml methylene chloride were added and methylene chloride evaporation was performed under a nitrogen stream (99.999 % grade). Medium (50/50 v/v acetonitrile/PBS) was added until reaching 4 ml in each test tube. The drug concentration was then estimated using HPLC.

An extraction factor was used for correction. Known weights of drug were dissolved in 3 ml acetonitrile and 3 ml PBS and 1 ml methylene chloride was added. The known concentrations were subjected to the same extraction procedure as the unknown concentrations in order to determine the efficiency of the extraction procedure.

The recovery efficiency of the method for FTS was 88.4 % and the value of the measured drug was corrected accordingly.

The recovery efficiency of the method for paclitaxel was 75 % and the value of the measured drug was corrected accordingly.

***Residual drug recovery from the drug-eluting fibers and stents:***

On the final day of the *in vitro* trial, residual FTS from composite drug-eluting fibers was measured as follows: the fibers were placed in 1 ml methylene chloride for 10 minutes and the coating shell was dissolved. 6 ml of a 50/50 acetonitrile/water solution were then added and the polyglyconate core was removed. Methylene chloride evaporation was performed under a nitrogen (99.999 %) stream. Medium (50/50 v/v acetonitrile/water) was added until 4 ml in each test tube and the FTS concentration was estimated by HPLC using the same method described above. An overall calibration curve for both HPLC and method recovery was calculated using known amounts of FTS under the same conditions.

The cumulative release profiles were determined relative to the initial amount of FTS in the composite fibers (quantity released during the incubation period plus the residue remaining in the fibers). All experiments were performed in triplicates.

Results are presented as means ± standard deviations. The effects of the emulsion's formulation on the release profile were studied by examining the following parameters: polymer content in the organic phase (% w/v), drug content relative to polymer content (% w/w), organic:aqueous (O:A) phase ratio and copolymer composition. The effect of the process kinetics (homogenization rate) on the release profile was also studied.

Residual FTS recovery from the fibers and stents was measured as follows: the stents were placed in methylene chloride (1 ml) and of a 50/50 % acetonitrile/water solution (6 ml) was added thereto. Methylene chloride evaporation was performed under nitrogen (99.999 %). Medium (50/50 % v/v acetonitrile/water) was added to reach 4 ml in each test tube and the FTS concentration was then determined using HPLC in the same method described above.

The cumulative release profiles were determined relative to the initial amount of FTS in the composite fibers (quantity released during the incubation period + the residue remaining in the fibers).

***In vitro degradation study of drug-eluting porous PDLGA films:***

Porous 50/50 % PDLGA film structures were fabricated using emulsion formulation containing 12.5 % w/v polymer in the organic solution, 2 % w/w FTS (relative to the polymer load), and an organic to aqueous (O:A) phase ratio of 4:1 v/v. The inverted emulsion was prepared as described hereinabove, poured into an aluminum plate and freeze dried in liquid nitrogen. Each sample was cut into parts of about 1 $cm^2$ in area and was incubated in 40 ml phosphate buffered saline (PBS) containing 0.05 % (v/v) sodium azide (as preservative) at 37 °C, under static conditions for 5 weeks. PBS was added when pH was out of range (between 7 and 8) or when the PBS volume dropped below 40 ml. Each film sample was taken out at weekly intervals and dried using a vacuum oven set at 35 °C for two hours, and stored in a dissector. The dried sample was immersed in methylene chloride to achieve a minimal concentration of about 0.13 % w/v.

The weight-average molecular weight and number-average molecular weight of the samples were determined by gel permeation chromatography (GPC). The GPC (Waters 21515 isocratic pump, operating temperature 40 °C using a column oven) was equipped with a refractive index detector (Waters 2414, operating temperature 40 °C) and calibrated with poly-L-lactic acid MW kit standards (Polysciences Inc., USA). Data were analyzed using the Breeze version 3.3 software. The samples were dissolved in methylene chloride, filtered, and eluted through 4 Styragel columns (model WAT044234 HR1 THF, WAT044237 HR2, WAT044225 HR4, WAT054460 HR5, 300 x 7.8 mm, 5 μm particle diameter) equipped with a guard column at a flow rate of 1 ml per minute, using Baker analyzed HPLC grade methylene chloride as eluent.

***Encapsulation efficiency studies:***

The encapsulation efficiency (EE) of the drug-eluting fibers was calculated as the actual amount ($M_a$) of drug measured in each fiber divided by the theoretical amount of drug ($M_t$) encapsulated during the fabrication process,

presented in percentage as shown in Equation 1 below.

$$\text{(Equation 1)} \qquad EE = \frac{M_a}{M_t}100$$

The actual amount of drug encapsulated within each fiber is the accumulated amount of FTS released at each measurement point of the trial plus the residual amount measured on day 35. The theoretical amount of FTS is the formulation drug concentration multiplied by the weight of the coating. The weight of the coating is the difference between the coated fiber (weighed at the beginning of the *in vitro* trial) and the bare fiber (weighed at the end of the trial, denuded of the coating by immersion in methylene chloride). The results are presented as means±standard deviations with n = 3 (triplicates).

***In vitro weight loss profile of the porous PDLGA structure:***

Porous 50/50 PDLGA and 75/25 film structures were fabricated as described hereinabove. Each film sample was cut into pieces of approximately 1 $cm^2$ and then incubated in 15 ml PBS at 37 °C and pH of 7.4 under static conditions. Samples (in triplicates) were taken out at weekly intervals, filtered using a 70 mm porcelain Büchner funnel equipped with a Whatman size 2 μm filtration paper and dried in a vacuum oven (35 °C for two hours).

Mass loss was measured using a Mettler-Toledo microbalance. The normalized mass loss was calculated by comparing the mass at a given time point ($w_t$) with the initial mass ($w_0$) as shown in Equation 2 below. The results are presented as means±standard deviations (n=3).

$$\text{(Equation 2)} \qquad Normalized\ \ weight = \frac{w_t}{w_0}100\%$$

***Measurements of water uptake:***

Porous 50/50 PDLGA film structures were fabricated as described hereinabove. Each film sample was cut into pieces of approximately 1 $cm^2$ and then incubated in 15 ml double-distilled water at 37 °C under static conditions. Samples (in triplicates) were taken out periodically and immediately subjected to measurement of wet weight, after surface water was removed with a clean-wipe tissue. Water uptake, namely adsorption and absorption of each sample during the swelling period, was determined according to Equation 3 below, wherein w is the wet weight at each time point and $w_0$ is the dry weight measured before the incubation.

$$\text{(Equation 3)} \qquad Water\ \ uptake = \frac{w - w_0}{w_0}100\%$$

***Measurements of tensile mechanical properties and degradation:***

Core-shell fiber structures were fabricated as described hereinabove. The fibers' tensile mechanical properties were measured at room temperature, under unidirectional tension at a rate of 50 mm/min, using a 5544 Instron uniaxel machine. Three fiber types (Maxon sutures, surface-treated fibers and coated fibers (without the drug), n=5 for each sample, 14 cm in length) were wrapped around a thick paper and inserted between the jigs. The tensile strength was defined as the maximum strength in the stress-strain curve. The maximal strain was defined as the breaking strain. Young's modulus was defined as the slope of the stress-strain curve in the elastic (linear) region. Means±standard deviations are presented.

In order to evaluate the degradation of mechanical properties, samples were immersed in 14 ml PBS filled tubes for 84 days. Each tube contained five specimens of 14 cm coated fibers. The pH was maintained between 7.3 and 7.5 and the medium was changed when the pH was out of the range. Each week a single tube was retrieved and the fibers were dried using a vacuum oven (35 °C for 1.5 hours) and kept in a desiccators. Each specimen's diameter was measured using a caliper and a tension test was carried out using an Instron machine as described hereinabove.

***Results:***

The dense core of the composite fibers presented herein enables obtaining the desired mechanical properties and the drug is located in a porous shell so as not to affect the mechanical properties. The shell is highly porous so as to enable release of the relatively hydrophobic antiproliferative drugs in a desired manner. In order to characterize the drug-eluting core/shell fiber or structure platform, FTS and paclitaxel, two exemplary drugs, were selected to study the release thereof from fibers (and stents in case of FTS) in light of the porpus morphology of the coating later and the degradation process.
The unique emulsion freeze drying technique presented herein was used to firm a porous coat over fibers and stents, such that preserve the temporal state of the emulsion in a solid form and the activity of the encapsulated drug. The coat is formed from inverted emulsions in which the continuous phase contained polymer and drug dissolved in a solvent, with water being the dispersed phase.
The effects of the inverted emulsion's parameters, i.e. polymer content, drug content, organic to aqueous (O:A) phase ratio and copolymer composition on the coat microstructure and on the drug release profile from the fibers, were presented in, for example, U.S. Patent Application having Publication No. 20070134305 by one of the present inventors. Optimal formulations were found for each exemplary drug (FTS or paclitaxel), which enabled to obtain a stable emulsion as may be inferred by the coat's bulk porous microstructure. Furthermore, in the current study 50/50 and 75/25 PDLGA were chosen as host polymers due to their relatively fast degradation rate in order to be able to release the hydrophobic antiproliferative agents at an appropriate rate.

***Microstructure of the coat of drug-eluting stents:***

Figure 9 presents a SEM micrograph of an FTS-eluting stent coated with PDLGA, prepared according to embodiments of the present invention, showing that only the struts of the stent are coated, leaving the openings between the struts free.
In order to estimate the coating's adhesion to the bare metal stent, a scalpel was used while the stent was immersed in liquid nitrogen in order to create a fracture in the coating. An example of one of these fractures is presented in Figure 10.
Figure 10 presents a SEM micrograph, showing an FTS-eluting stent having a fracture in the coating, exposing the stent-coating interface and showing the porous micro-structure of the coat.
As can be seen in Figure 10, there are no gaps between the coating and the stent, indicating complete and uniform adhesion of the PDLGA coating to the metal.
The shell's porous structure in all studied specimens based on stable emulsions contained round-shaped pores having an average diameter of $2.42 \pm 0.68$ μm, with a porosity level of $51.30 \pm 9.11$ %. The shell's microstructure was uniform in each sample due to the rapid freezing of the inverted emulsion, which enabled preservation of its microstructure. The pores were partially interconnected by smaller inner pores. The microstructure of the coat affects the drug release profile. The high porosity, small pores, and partially interconnectivity of the inner pores in the stent coating, can be controlled by the emulsion's formulation and the process parameters.

***Cumulative drug-release profiles from drug-eluting stents:***

Figure 11 presents comparative plots of the cumulative drug-release profiles of two PDLGA coated FTS-eluting stents according to embodiments of the present invention, measured over four weeks (28 days), showing a mean overall release of $53.95 \pm 9.73$ μg FTS (results are presented as means $\pm$ standard deviation).
Figure 12 presents comparative plots of the normalized accumulated FTS-release profiles of two PDLGA coated FTS-eluting stents according to embodiments of the present invention, showing a mean of $81.38 \pm 10.88$ % of the total encapsulated FTS released over a period of 28 days and the mean initial burst release of $37.23 \pm 7.47$ % during the first day of the experiment (results are presented as means $\pm$ standard deviation).
Figure 13 presents a plot of the average molecular weight of the PDLGA coating as a function of time, representing the degradation profile of the porous PDLGA coating, and showing that the rate of degradation during the first 16 days is higher than in the following days (error bars present standard deviation, n=3). It is assumed that during the first phase of FTS release (14 days) diffusion is the dominant mechanism for drug release, while during the second phase of release the massive degradation of the host polymer (already achieved) has a significant contribution to drug release form the porous coating.
Figure 14 presents a photograph of the FTS-eluting stent after 28 days of incubation in PBS medium. As can be seen in Figure 14, the coating seems intact and adherent to the stent's struts although massive degradation leading to erosion (weight loss) of the polymer has already occurred at this stage.
As can be concluded from the above results, the drug-eluting stents according to some embodiments of the

present invention, coated with PDLGA/FTS, exhibited adequate adhesion of the porous coating to the metal surface of the stent. The FTS-release profile from the stent showed a burst effect followed by a moderate release profile. Approximately 80 % of the encapsulated drug was released within four weeks, as desired for this application. Degradation of the host polymer controls the rate of release of the drug.

In general, the porous coat structures (porosity of 67-85 % and pore size of 2-7 μm) exhibited a relatively fast FTS release within several weeks and a slower paclitaxel release within several months. The copolymer composition was found to be an important parameter affecting release behavior in the systems presented herein. Its effect can be described as follows: an increase in the glycolic acid content of the PDLGA copolymer resulted in an increase in the burst effect and release rate of FTS during the first two weeks, mainly due to higher water uptake, swelling and changes in microstructure. Higher glycolic acid also enabled faster paclitaxel release, mainly due to a faster degradation rate of the host polymer. In addition, an indirect effect of the microstructure on the release profile occurs via an emulsion stability mechanism, i.e. a higher diffusion rate of the hydrophobic antiproliferative agents can be achieved when high porosity is combined with a fine structure of lower pore size. The direct effect is more significant than the indirect effect.

Although the invention has been described in conjunction with specific embodiments thereof, it is evident that many alternatives, modifications and variations will be apparent to those skilled in the art. Accordingly, it is intended to embrace all such alternatives, modifications and variations that fall within the broad scope of the appended claims.

Any citation or identification of any reference in this application shall not be construed as an admission that such reference is available as prior art to the present invention. To the extent that section headings are used, they should not be construed as necessarily limiting.

***References cited by numerals***

***(Other references are cited in the text)***

1. Tamai, H., et al., Circulation, 2000. 102(4): p. 399-404.
2. Tamai, H., et al., J. Intervent. Cardiol., 1999. 12: p. 443-449.
3. Virmani, R., et al., Circulation, 2004. 109(6): p. 701-5.
4. Revell, P. A., et al., Biomaterials, 1998. 19(17): p. 1579-86.
5. Serruys, P. W., et al., J Am Coll Cardiol, 2005. 46(2): p. 253-60.
6. Carter, A. J., et al., Cardiovasc Res, 2004. 63(4): p. 617-24.
7. Wang, X., et al., Biomaterials, 2006. 27(32): p. 5588-95.
8. Venkatraman, S., et al., J Control Release, 2007. 120(3): p. 149-60.
9. Colombo, A., et al., Circulation, 2003. 108(7): p. 788-94.
10. Drachman, D. E., et al., J Am Coll Cardiol, 2000. 36(7): p. 2325-32.
11. Finkelstein, A., et al., Circulation, 2003. 107(5): p. 777-84.
12. Westedt, U., et al., J Control Release, 2006. 111(1-2): p. 235-46.
13. Breitenbach, A., et al., J Control Release, 2000. 63(1-2): p. 53-68.
14. Galdbart, J. O., et al., J Surg Res, 1996. 66(2): p. 174-8.
15. Strachan, C. J., et al., Eur J Vasc Surg, 1991. 5(6): p. 627-32.

**Claims**

1. A composite structure comprising a device and at least one polymeric porous coat coating at least a part of said device and encapsulating at least one bioactive agent, said coat encapsulating said at least one bioactive agent while retaining an activity of said bioactive agent and/or while releasing said bioactive agent in a pre-determined release rate, the composite structure being prepared by contacting said device and a water-in-oil emulsion containing an aqueous solution and an organic solution, said organic solution containing at least one second polymer and said emulsion further containing said at least one bioactive agent either within said aqueous solution or within said organic solution, to thereby obtain said device having a layer of said emulsion applied on at least a part thereof, and by freeze-drying said device having said layer applied thereon,
with the proviso that said device is not a fiber and further with the proviso that said device is comprised of fibrous elements, said coat is not coating said fibrous elements at the contact point of intercrossing junctions of said fibrous elements in said device, such that said fibrous elements are in contact with each other in each of said junctions.

2. The composite structure of claim 1, wherein said polymeric coat comprises a polymer selected from the group

consisting of an aliphatic polyester made of glycolide (glycolic acid), lactide (lactic acid), poly(DL-lactic-co-glycolic acid), caprolactone, p-dioxanone, trimethylene carbonate, hydroxybutyrate, and/or hydroxyvalerate; a polypeptide made of natural and modified amino acids; a polyether made of at least one natural and modified saccharide; a polydepsipeptide; a biodegradable nylon co-polyamide; a polydihydropyran, a polyphosphazene, a poly(ortho-ester), a poly(cyanoacrylate), a polyanhydride, poly(glycolic acid), poly(lactic acid), polydioxanone (PDS), poly(alkylene succinate), poly(hydroxybutyrate), poly(butylene diglycolate), poly(epsilon-caprolactone), and any copolymer thereof.

**3.** The composite structure of claim 1, wherein said device is a medical device.

**4.** The composite structure of any of claims 1-3, wherein said device is a medical device selected from the group consisting of a mesh, a suture mesh, a wound dressing, a stent, a skin patch, a bandage, a suture anchor, a screw, a pin, a tack, a rod, an angioplastic plug, a plate, a clip, a ring, a needle, a tube, a dental implant, an orthopedic implant, a guided tissue matrix, an aortic aneurysm graft device, an atrioventricular shunt, a catheter, a heart valve, a hemodialysis catheter, a bone-fracture healing device, a bone replacement device, a joint replacement device, a tissue regeneration device, a tumor targeting and destruction device, a periodontal device, a hernia repair device, a hemodialysis graft, an indwelling arterial catheter, an indwelling venous catheter, a pacemaker casing, a pacemaker lead, a patent foramen ovale septal closure device, a vascular stent, a tracheal stent, an esophageal stent, a urethral stent, a rectal stent, a stent graft, a synthetic vascular graft, a vascular aneurysm occluder, a vascular clip, a vascular prosthetic filter, a vascular sheath, a drug delivery port and a venous valve.

**5.** The composite structure of any of claims 1-4, wherein said device has a mesh structure.

**6.** The composite structure of any of claims 1-5, wherein said polymeric coat is **characterized by** a pore density that ranges from 5 % of void volume per coat volume to 95 % of void volume per coat volume.

**7.** The composite structure of claim 1, wherein said device is a stent device and said at least one bioactive agent is selected from the group consisting of paclitaxel, sirolimus, everolimus, zotarolimus, farnesylthiosalicylate (FTS) and fluoro-FTS.

**8.** The composite structure of claim 1, wherein said bioactive agent is a farnesyl derivative having the general Formula I:

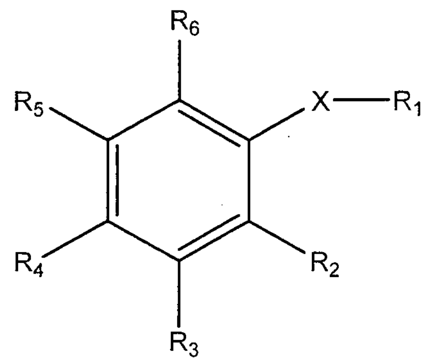

Formula I

wherein:

$R_1$ is selected from the group consisting of farnesyl, geranyl or geranyl-geranyl;
$R_2$ is selected from the group consisting of hydrogen, -C≡N, $-COOR_7$, $-SO_3R_7$, $-CONR_7R_8$ and $SO_2NR_7R_8$, -COOM and $-SO_3M$;
$R_7$ and $R_8$ are each independently selected from the group consisting of hydrogen, alkyl and alkenyl;
M is a cation;
$R_3$, $R_4$, $R_5$ and $R_6$ are each independently selected from the group consisting of hydrogen, carboxyl, alkyl, alkenyl, aminoalkyl, nitroalkyl, nitro, halo, amino, mono- or di-alkylamino, mercapto, mercaptoalkyl, azido, or thiocyanato;
X is selected from the group consisting of O, S, SO, $SO_2$, NH or Se,

the composite structure being a drug-eluting stent.

9. The composite structure of claim 1, wherein said device is a mesh device and said bioactive agent is an antimicrobial agent, the composite structure being a drug-eluting mesh.

10. A process of preparing a composite structure which comprises a device and a porous polymeric coat coating at least a part of said device, wherein the coat comprises at least one bioactive agent encapsulated therein and/or applied thereon, the process comprising:

contacting said device and a water-in-oil emulsion containing an aqueous solution and an organic solution, said organic solution containing at least one second polymer and said emulsion further containing said at least one bioactive agent either within said aqueous solution or within said organic solution, to thereby obtain said device having a layer of said emulsion applied on at least a part thereof; and

freeze-drying said device having said layer applied thereon, thereby obtaining the composite structure;

with the proviso that said device is not a fiber and further with the proviso that when said device is comprised of fibrous elements, said layer of said emulsion is not applied on said fibrous elements at the contact point of intercrossing junctions of said fibrous elements in said device, such that said fibrous elements are in contact with each other in each of said junctions.

11. The composite structure of claim 7, wherein said farnesyl derivative is farnesylthiosalicylate (FTS) or fluoro-FTS.

12. The composite structure of claim 7, wherein said polymeric porous coat comprises poly(DL-lactic-co-glycolic acid).

13. The composite structure of claim 9, wherein said antimicrobial agent is selected from the group consisting of gentamicin, ceftazidime and mafenide.

14. The composite structure of claim 8, wherein said polymeric porous coat comprises poly(DL-lactic-co-glycolic acid).

## Patentansprüche

1. Verbundstruktur, eine Vorrichtung und mindestens einen porösen Polymerüberzug umfassend, der zumindest einen Teil der Vorrichtung überzieht und mindestens ein bioaktives Mittel einkapselt, wobei der Überzug das mindestens eine bioaktive Mittel einkapselt, während eine Wirksamkeit des bioaktiven Mittels bewahrt wird und/oder während das bioaktive Mittel in einer zuvor festgelegten Freisetzungsrate freigesetzt wird, wobei die Verbundstruktur hergestellt wird, indem die Vorrichtung und eine Wasser-in-Öl-Emulsion, die eine wässrige Lösung und eine organische Lösung enthält, in Kontakt gebracht werden, wobei die organische Lösung mindestens ein zweites Polymer enthält und die Emulsion ferner mindestens ein bioaktives Mittel enthält, entweder in der wässrigen Lösung oder in der organischen Lösung, um dadurch zu erzielen, dass die Vorrichtung eine Schicht aus der Emulsion aufweist, die auf zumindest einem Teil von ihr aufgebracht ist, und indem die Vorrichtung mit der darauf aufgebrachten Schicht gefriergetrocknet wird,

unter der Voraussetzung, dass die Vorrichtung keine Faser ist, und ferner unter der Voraussetzung, dass, wenn die Vorrichtung aus fibrösen Elementen besteht, der Überzug nicht die fibrösen Elemente am Kontaktpunkt von Kreuzverbindungen der fibrösen Elemente in der Vorrichtung überzieht, so dass die fibrösen Elemente an jeder der Verbindungen in Kontakt miteinander stehen.

2. Verbundstruktur nach Anspruch 1, wobei der Polymerüberzug ein Polymer umfasst, das aus der Gruppe ausgewählt ist, die besteht aus: einem aliphatischen Polyester, bestehend aus Glycolid (Glycolsäure), Lactid (Milchsäure), Poly(DL-Milch-co-Glycolsäure), Caprolacton, p-Dioxanon, Trimethylencarbonat, Hydroxybutyrat und/oder Hydroxyvalerat; einem Polypeptid, bestehend aus natürlichen oder modifizierten Aminosäuren; einem Polyether, bestehend aus mindestens einem natürlichen und modifizierten Saccharid; einem Polydepsipeptid; einen biologisch abbaubaren Nylon-coPolyamid; einem Polydihydropyran, einem Polyphosphazen, einem Poly(ortho-ester), einem Poly(cyanoacrylat), einem Polyanhydrid, Poly(glycolsäure), Poly(milchsäure), Polydioxanon (PDS), Poly(alkylensuccinat), Poly(hydroxybutyrat), Poly(butylendiglycolat), Poly(epsilon-caprolacton) und einem Copolymer daraus.

3. Verbundstruktur nach Anspruch 1, wobei die Vorrichtung eine medizinische Vorrichtung ist.

4. Verbundstruktur nach einem der Ansprüche 1 bis 3, wobei die Vorrichtung eine medizinische Vorrichtung ist, die

aus der Gruppe ausgewählt ist, bestehend aus: einem Netz, einem chirurgischen Netz, einem Wundverband, einem Stent, einem Hautpflaster, einer Bandage, einem Fadenanker, einer Schraube, einem Stift, einer Heftklammer, einem Stab, einem Angioplastie-Tamponadepfropf, einer Platte, einer Klammer, einem Ring, einer Nadel, einer Röhre, einem Dentalimplantat, einem orthopädischen Implantat, einer gesteuerten Gewebematrix, einem Aortenaneurysmatransplantat, einem arteriovenösen Shunt, einem Katheter, einer Herzklappe, einem Hämodialysekatheter, einer Knochenbruch-Heilungsvorrichtung, einer Knochenersatzvorrichtung, einer Gelenkersatzvorrichtung, einer Geweberegenerationsvorrichtung, einer tumortagetierenden und -zerstörenden Vorrichtung, einer Paradontalvorrichtung, einer Hernienreparaturvorrichtung, einerm Hämodialysetransplantat, einem Arterienverweilkatheter, einem Venenverweilkatheter, einem Schrittmachergehäuse, einer Schrittmachersonde, einer Verschlussvorrichtung für ein offenes Foramen ovale, einem Gefäß-Stent, einem Luftröhren-Stent, einem Speiseröhren-Stent, einem Harnröhren-Stent, einem Rektal-Stent, einem Stent-Transplantat, einem synthetischen Gefäßtransplantat, einem Gefäßaneurysmaokkluder, einer Gefäßklammer, einem Gefäßprothesefilter, einer Gefäßscheide, einem Arzneimittelzufuhranschluss und einer Venenklappe.

5. Verbundstruktur nach einem der Ansprüche 1 bis 4, wobei die Vorrichtung eine Netzstruktur aufweist.

6. Verbundstruktur nach einem der Ansprüche 1 bis 5, wobei der Polymerüberzug durch eine Porendichte im Bereich von 5 % Porenvolumen pro Überzugvolumen bis 95 % Porenvolumen pro Überzugvolumen gekennzeichnet ist.

7. Verbundstruktur nach Anspruch 1, wobei die Vorrichtung eine Stent-Vorrichtung ist und das mindestens eine bioaktive Mittel aus der Gruppe ausgewählt ist, die aus Paclitaxel, Sirolimus, Everolimus, Zotarolirnus, Farnesylthiosalicylat (FTS) und Fluor-FTS besteht.

8. Verbundstruktur nach Anspruch 1, wobei das bioaktive Mittel ein Farnesylderivat mit der allgemeinen Formel I ist:

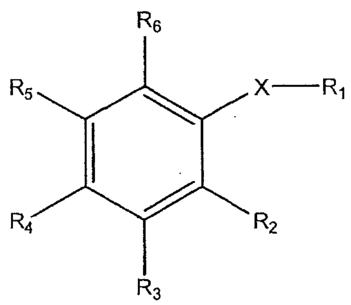

Formel I

wobei:

$R_1$ aus der Gruppe ausgewählt ist, die aus Farnesyl, Geranyl oder Geranyl-Geranyl besteht;
$R_2$ aus der Gruppe ausgewählt ist, die aus Wasserstoff, $-C\equiv N$, $-COOR_7$, $-SO_3R_7$,$-CONR_7R_8$ und $SO_2NR_7R_8$, $-COOM$ und $-SO_3M$ besteht;
$R_7$ und $R_8$ jeweils unabhängig aus der Gruppe ausgewählt sind, die aus Wasserstoff, Alkyl und Alkenyl besteht;
M ein Kation ist;
$R_3$, $R_4$, $R_5$ und $R_6$ jeweils unabhängig aus der Gruppe ausgewählt sind, die aus Wasserstoff, Carboxyl, Alkyl, Alkenyl, Aminoalkyl, Nitroalkyl, Nitro, Halo, Amino, Mono- oder Dialkylamino, Mercapto, Mercaptoalkyl, Azido oder Thiocyanato besteht;
X aus der Gruppe ausgewählt ist, die aus O, S, SO, $SO_2$, NH oder Se besteht,
wobei die Verbundstruktur ein arzneimittel-eluierender Stent ist.

9. Verbundstruktur nach Anspruch 1, wobei die Vorrichtung eine Netzvorrichtung ist und das bioaktive Mittel ein antimikrobielles Mittel ist, wobei die Verbundstruktur ein arzneimittel-eluierender Stent ist.

10. Verfahren zur Herstellung einer Verbundstruktur, die eine Vorrichtung und einen porösen Polymerüberzug umfasst, der zumindest einen Teil der Vorrichtung überzieht, wobei der Überzug mindestens ein bioaktives Mittel umfasst, das in ihm eingekapselt und/oder auf ihn aufgebracht wird, wobei das Verfahren Folgendes umfasst:

In-Kontakt-Bringen der Vorrichtung und einer Wasser-in-Öl-Emulsion, die eine wässrige Lösung und eine organische Lösung enthält, wobei die organische Lösung mindestens ein zweites Polymer enthält und die Emulsion ferner mindestens ein bioaktives Mittel enthält, entweder in der wässrigen Lösung oder in der organischen Lösung, um dadurch zu erzielen, dass die Vorrichtung eine Schicht aus der Emulsion aufweist, die auf zumindest einem Teil von ihr aufgebracht ist, und

Gefriertrocknen der Vorrichtung mit der darauf aufgebrachten Schicht, wodurch die Verbundstruktur erzielt wird, unter der Voraussetzung, dass die Vorrichtung keine Faser ist, und ferner unter der Voraussetzung, dass, wenn die Vorrichtung aus fibrösen Elementen besteht, die Schicht aus der Emulsion nicht am Kontaktpunkt von Kreuzverbindungen der fibrösen Elemente in der Vorrichtung auf die fibrösen Elemente aufgebracht wird, so dass die fibrösen Elemente an jeder der Verbindungen in Kontakt miteinander stehen.

**11.** Verbundstruktur nach Anspruch 7, wobei das Farnesylderivat Farnesylthiosalicylat (FTS) oder Fluor-FTS ist.

**12.** Verbundstruktur nach Anspruch 7, wobei der poröse Polymerüberzug Poly(DL-Milch-co-Glycolsäure) umfasst.

**13.** Verbundstruktur nach Anspruch 9, wobei das antimikrobielle Mittel aus der Gruppe ausgewählt ist, die aus Gentamicin, Ceftazidim und Mafenid besteht.

**14.** Verbundstruktur nach Anspruch 8, wobei der poröse Polymerüberzug Poly(DL-Milch-co-Glycolsäure) umfasst.

## Revendications

**1.** Structure composite comprenant un dispositif et au moins un revêtement poreux polymère revêtant au moins une partie dudit dispositif et encapsulant au moins un agent bioactif, ledit revêtement encapsulant ledit au moins un agent bioactif tout en conservant une activité dudit agent bioactif et/ou tout en libérant ledit agent bioactif à un taux de libération prédéterminé, la structure composite étant préparée en mettant en contact ledit dispositif et une émulsion d'eau dans l'huile contenant une solution aqueuse et une solution organique, ladite solution organique contenant au moins un second polymère et ladite émulsion contenant en outre ledit au moins un agent bioactif soit à l'intérieur de ladite solution aqueuse soit à l'intérieur de ladite solution organique, pour ainsi obtenir ledit dispositif ayant une couche de ladite émulsion appliquée sur au moins une partie de celui-ci, et en lyophilisant ledit dispositif sur lequel ladite couche est appliquée,

à condition que ledit dispositif ne soit pas une fibre et à la condition supplémentaire que lorsque ledit dispositif est composé d'éléments fibreux, ledit revêtement ne revête pas lesdits éléments fibreux au point de contact des jonctions d'entrecroisement desdits éléments fibreux dans ledit dispositif, de sorte que lesdits éléments fibreux soient en contact les uns avec les autres dans chacune desdites jonctions.

**2.** Structure composite selon la revendication 1, dans laquelle ledit revêtement polymère comprend un polymère choisi dans le groupe constitué d'un polyester aliphatique fabriqué à partir de glycolide (acide glycolique), lactide (acide lactique), poly(acide DL-lactique-co-glycolique), caprolactone, p-dioxanone, carbonate de triméthylène, hydroxybutyrate et/ou d'hydroxyvalérate ; d'un polypeptide fabriqué à partir d'acides aminés naturels et modifiés ; d'un polyéther fabriqué à partir d'au moins un saccharide naturel et modifié; d'un polydepsipeptide ; d'un nylon co-polyamide biodégradable; d'un polydihydropyrane, d'un polyphosphazène, d'un poly(ortho-ester), d'un poly(cyanoacrylate), d'un polyanhydride, d'un poly(acide glycolique), d'un acide poly(acide lactique), d'une polydioxanone (PDS), d'un poly(succinate d'alkylène), d'un poly(hydroxybutyrate), d'un poly(diglycolate de butylène), d'une poly(epsilon-caprolactone), et de tout copolymère de ceux-ci.

**3.** Structure composite selon la revendication 1, dans laquelle ledit dispositif est un dispositif médical.

**4.** Structure composite selon l'une quelconque des revendications 1 à 3, dans laquelle ledit dispositif est un dispositif médical choisi dans le groupe constitué d'une maille, d'une maille de suture, d'un pansement, d'une endoprothèse, d'un timbre cutané, d'un bandage, d'une ancre de suture, d'une vis, d'une broche, d'une broquette, d'une tige, d'un bouchon angioplastique, d'une plaque, d'un clip, d'un anneau, d'une aiguille, d'un tube, d'un implant dentaire, d'un implant orthopédique, d'une matrice tissulaire guidée, d'un dispositif de greffe pour anévrisme aortique, d'une dérivation atrioventriculaire, d'un cathéter, d'une valvule cardiaque, d'un cathéter d'hémodialyse, d'un dispositif destiné à la guérison d'une fracture osseuse, d'un dispositif de remplacement osseux, d'un dispositif de remplacement articulaire, d'un dispositif de régénération tissulaire, d'un dispositif de ciblage et de destruction de tumeur, d'un dispositif périodontal, d'un dispositif de réparation de hernie, d'une greffe d'hémodialyse, d'un cathéter artériel à

demeure, d'un cathéter veineux à demeure, d'un boîtier de stimulateur cardiaque, d'un fil de stimulateur cardiaque, d'un dispositif de fermeture d'orifice septal pour persistance du foramen ovale, d'une endoprothèse vasculaire, d'une endoprothèse trachéale, d'une endoprothèse oesophagique, d'une endoprothèse urétrale, d'une endoprothèse rectale, d'une greffe d'endoprothèse, d'un greffon vasculaire synthétique, d'un dispositif d'occlusion d'anévrisme vasculaire, d'un clip vasculaire, d'un filtre prothétique vasculaire, d'une gaine vasculaire, d'un orifice d'administration de médicament et d'une valvule veineuse.

**5.** Structure composite selon l'une quelconque des revendications 1 à 4, dans laquelle ledit dispositif a une structure maillée.

**6.** Structure composite selon l'une quelconque des revendications 1 à 5, dans laquelle ledit revêtement polymère est **caractérisé par** une densité de pores qui se situe dans la plage de 5 % de volume de vide par volume de revêtement à 95 % de volume de vide par volume de revêtement.

**7.** Structure composite selon la revendication 1, dans laquelle ledit dispositif est un dispositif formant endoprothèse et ledit au moins un agent bioactif est choisi dans le groupe constitué du paclitaxel, du sirolimus, de l'évérolimus, du zotarolimus, du farnésylthiosalicylate (FTS) et du fluoro-FTS.

**8.** Structure composite selon la revendication 1, dans laquelle ledit agent bioactif est un dérivé du farnésyle répondant à la Formule générale I :

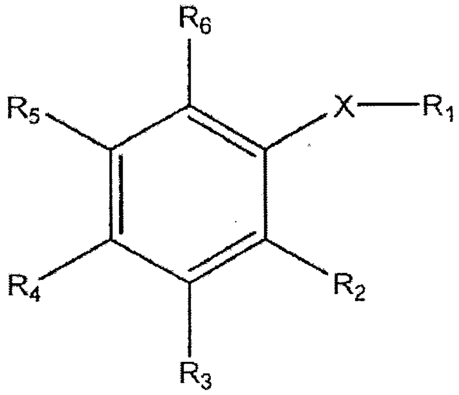

Formula I

dans laquelle :

$R_1$ est choisi dans le groupe constitué des groupes farnésyle, géranyle ou géranyl-géranyle ;
$R_2$ est choisi dans le groupe constitué de l'atome d'hydrogène et des groupes -C≡N, - $COOR_7$, $-SO_3R_7$, $-CONR_7R_8$ et $SO_2NR_7R_8$, -COOM et $-SO_3M$ ;
$R_7$ et $R_8$ sont chacun indépendamment choisis dans le groupe constitué de l'atome d'hydrogène et des groupes alkyle et alcényle ;
M est un cation ;
$R_3$, $R_4$, $R_5$ et $R_6$ sont chacun indépendamment choisis dans le groupe constitué de l'atome d'hydrogène et des groupes carboxyle, alkyle, alcényle, aminoalkyle, nitroalkyle, nitro, halogéno, amino, mono- ou di-alkylamino, mercapto, mercaptoalkyle, azido ou thiocyanato ;
X est choisi dans le groupe constitué de l'atome d'O, de l'atome de S, des groupes SO, $SO_2$, NH ou de l'atome de Se,
la structure composite étant une endoprothèse à élution médicamenteuse.

**9.** Structure composite selon la revendication 1, dans laquelle ledit dispositif est un dispositif maillé et ledit agent bioactif est un agent antimicrobien, la structure composite étant une maille à élution médicamenteuse.

**10.** Procédé de préparation d'une structure composite qui comprend un dispositif et un revêtement polymère poreux revêtant au moins une partie dudit dispositif, dans lequel le revêtement comprend au moins un agent bioactif encapsulé dans celui-ci et/ou appliqué sur celui-ci, le procédé comprenant :

la mise en contact dudit dispositif et d'une émulsion d'eau dans l'huile contenant une solution aqueuse et une solution organique, ladite solution organique contenant au moins un second polymère et ladite émulsion contenant en outre ledit au moins un agent bioactif soit à l'intérieur de ladite solution aqueuse soit à l'intérieur de ladite solution organique, pour ainsi obtenir ledit dispositif ayant une couche de ladite émulsion appliquée sur au moins une partie de celui-ci ; et
la lyophilisation dudit dispositif sur lequel ladite couche est appliquée, pour ainsi obtenir la structure composite ;
à condition que ledit dispositif ne soit pas une fibre et à la condition supplémentaire que lorsque ledit dispositif est composé d'éléments fibreux, ladite couche de ladite émulsion ne soit pas appliquée sur lesdits éléments fibreux au point de contact des jonctions d'entrecroisement desdits éléments fibreux dans ledit dispositif, de sorte que lesdits éléments fibreux soient en contact les uns avec les autres dans chacune desdites jonctions.

**11.** Structure composite selon la revendication 7, dans laquelle ledit dérivé du farnésyle est du farnésylthiosalicylate (FTS) ou du fluoro-FTS.

**12.** Structure composite selon la revendication 7, dans laquelle ledit revêtement poreux polymère comprend du poly(acide DL-lactique-co-glycolique).

**13.** Structure composite selon la revendication 9,
dans laquelle ledit agent antimicrobien est choisi dans le groupe constitué de la gentamicine, de la ceftazidime et du mafénide.

**14.** Structure composite selon la revendication 8, dans laquelle ledit revêtement poreux polymère comprend du poly(acide DL-lactique-co-glycolique).

FIG. 1

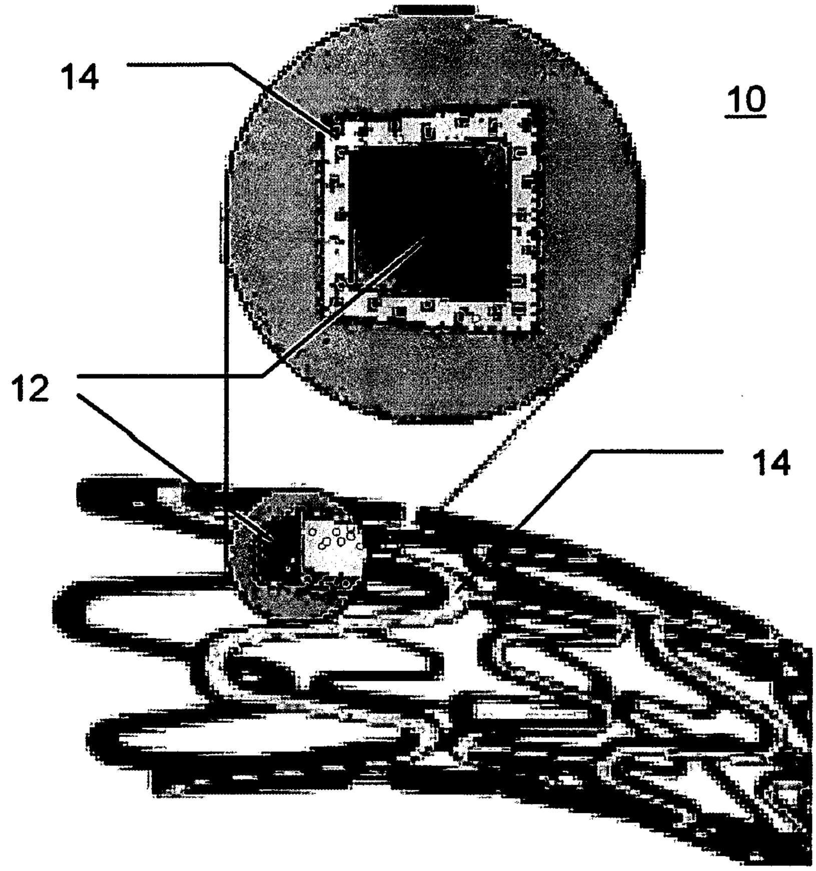


FIG. 2A

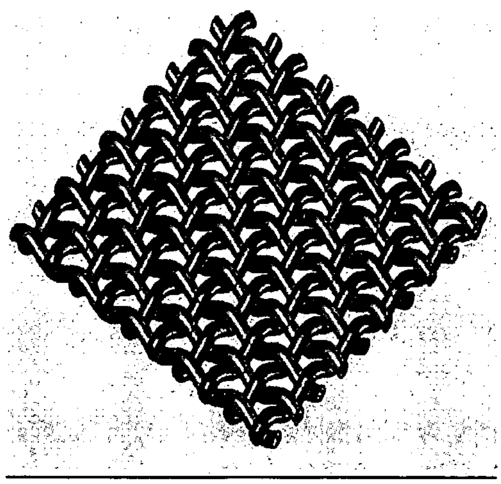

FIG. 2B

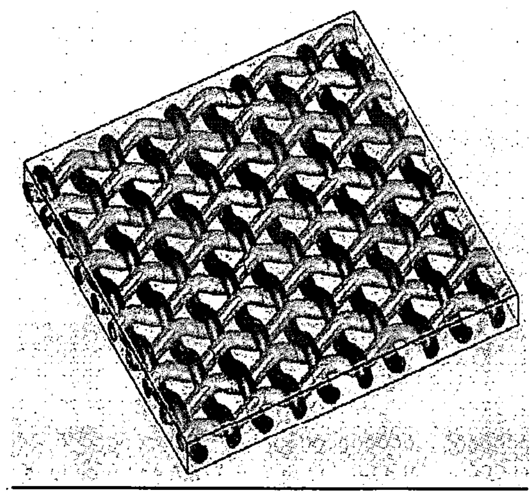

FIG. 3A

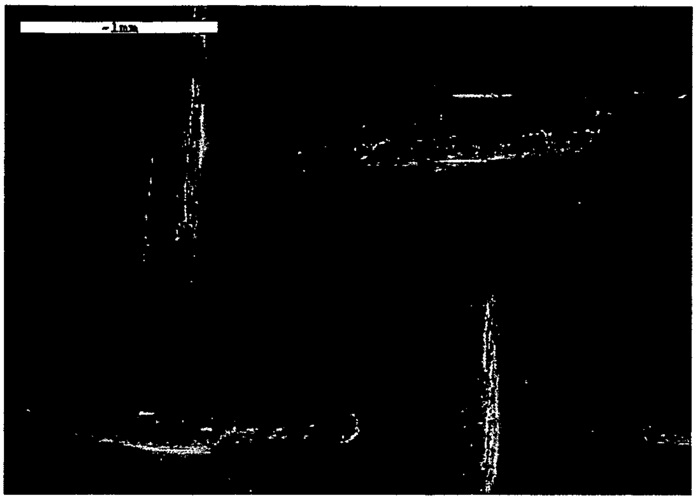


FIG. 3B

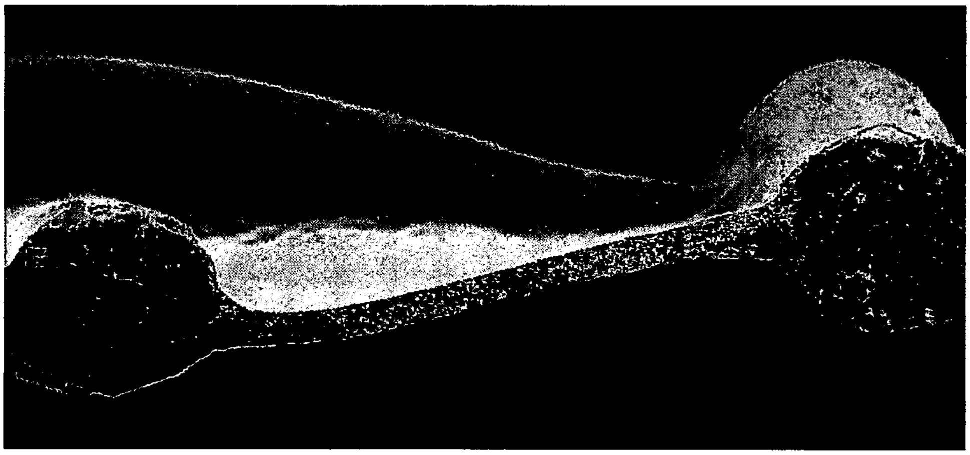

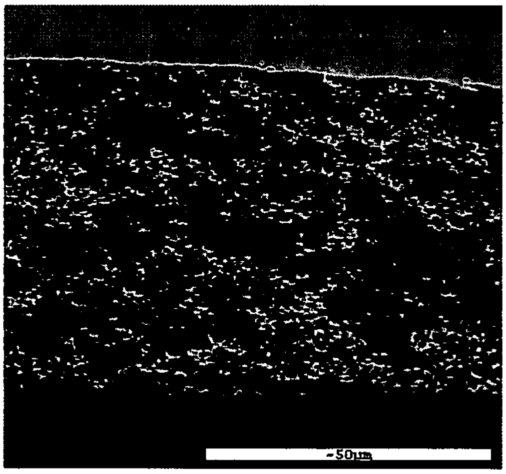


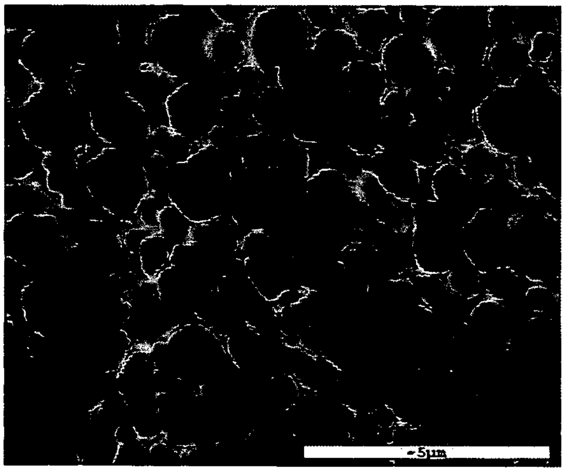


FIG. 3C

FIG. 3D

FIG. 4A

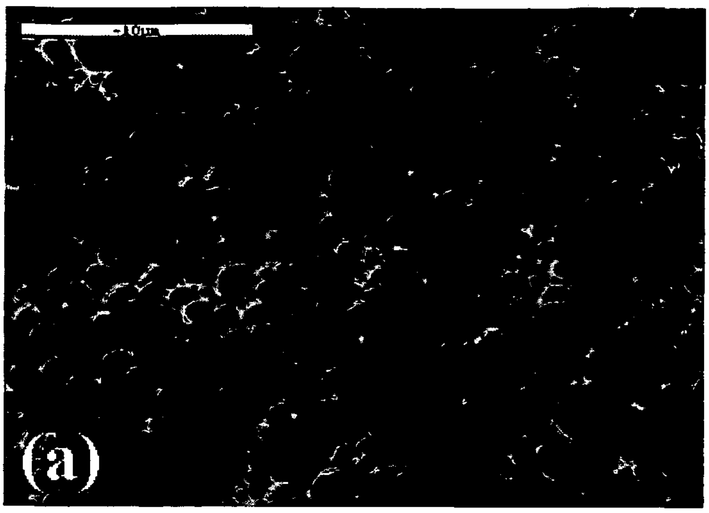


FIG. 4B

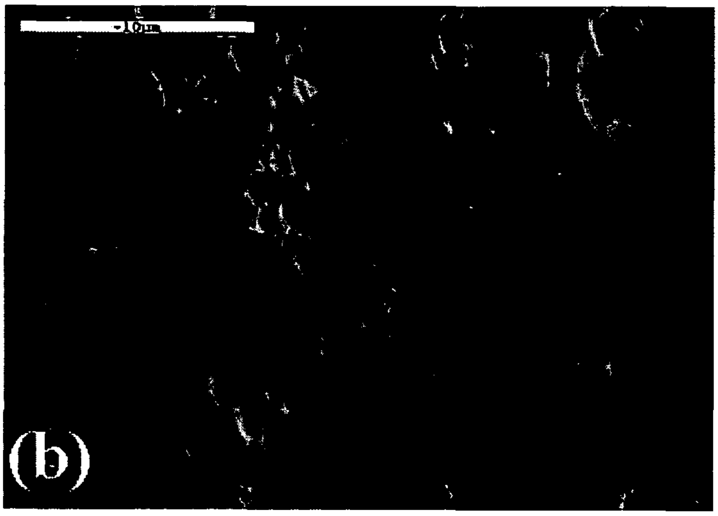


FIG. 4C

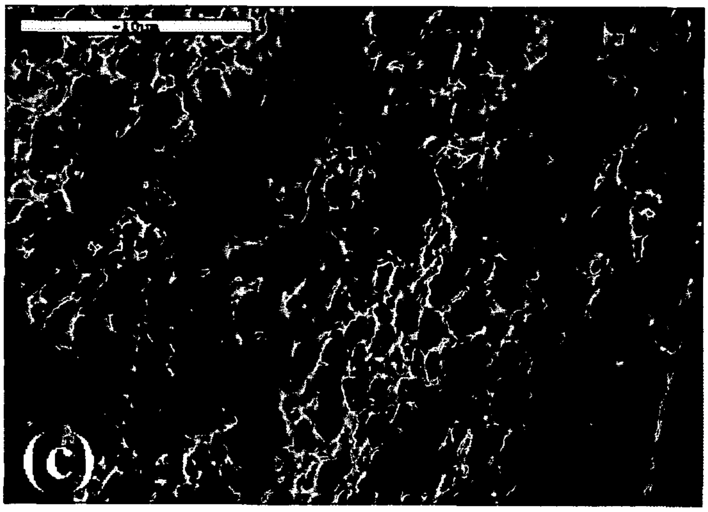

FIG. 5A

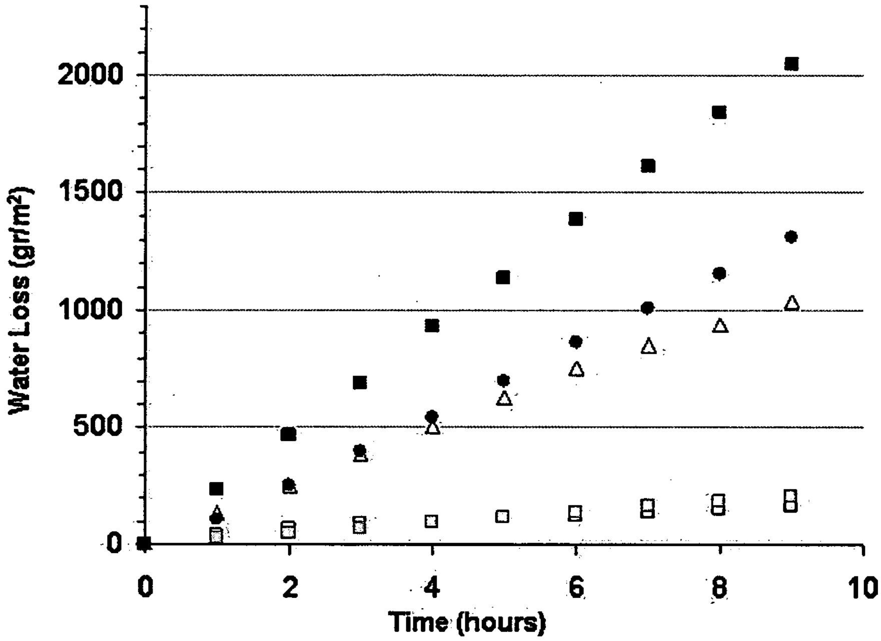
2000
1500
1000
500
0
Water Loss (gr/m$^2$)
0
2
4
6
8
10
Time (hours)

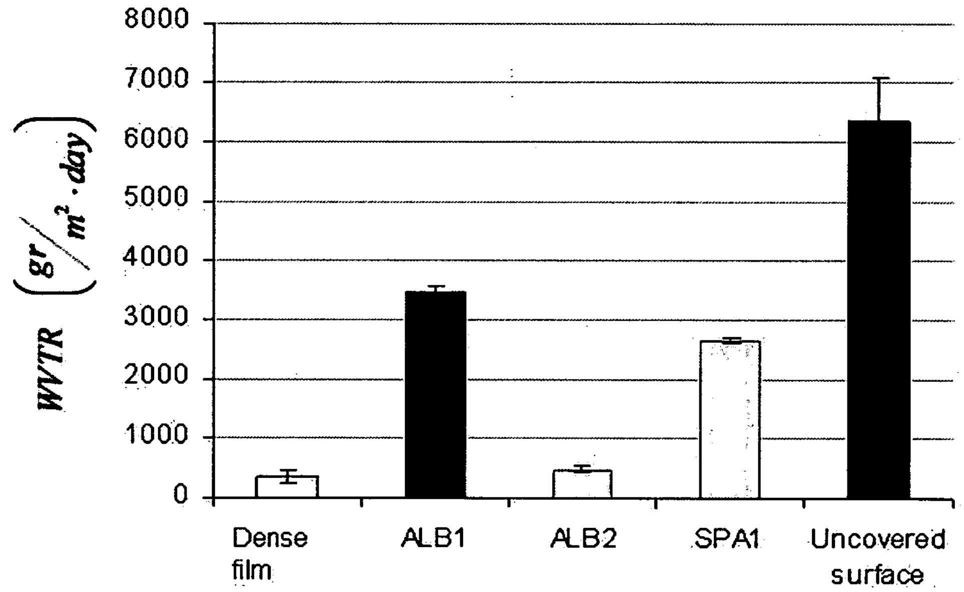
8000
7000
6000
5000
4000
3000
2000
1000
0
WVTR (gr/m$^2$·day)
Dense film
ALB1
ALB2
SPA1
Uncovered surface

FIG. 5B

FIG. 6A

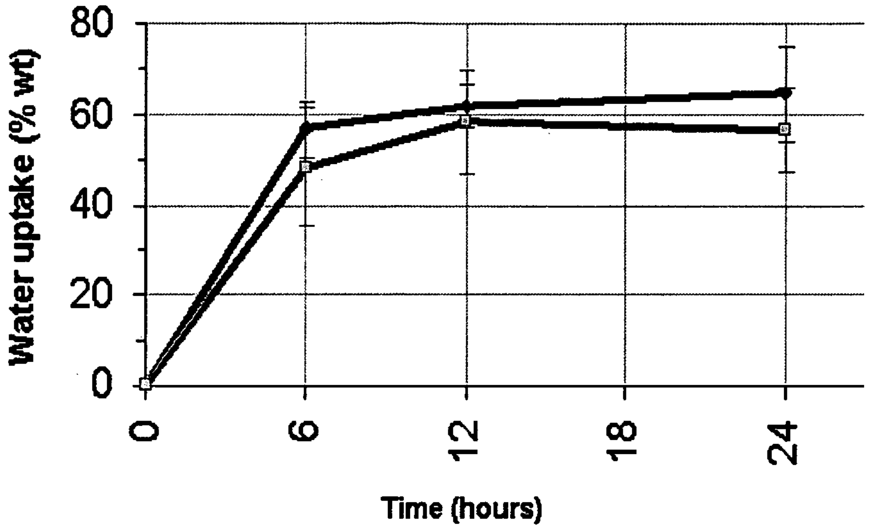
80
60
40
20
0
Water uptake (% wt)
0
6
12
18
24
Time (hours)

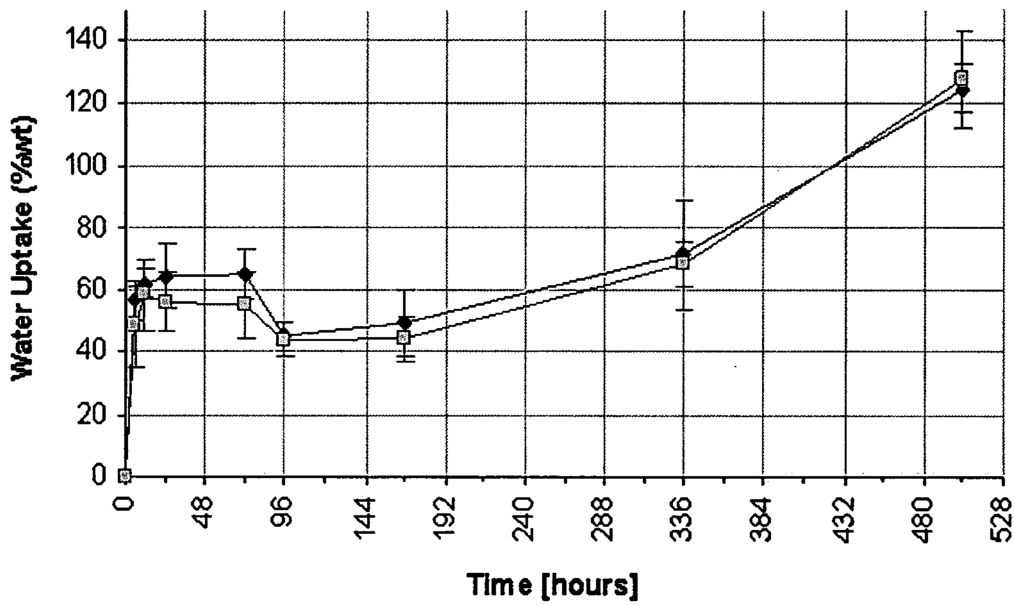
140
120
100
80
60
40
20
0
Water Uptake (%wt)
0
48
96
144
192
240
288
336
384
432
480
528
Time [hours]

FIG. 6B

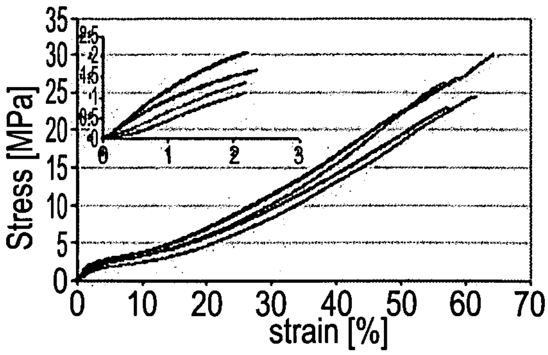


**FIG. 7A**

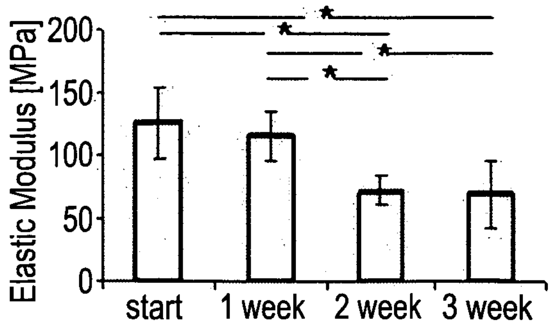


**FIG. 7B**

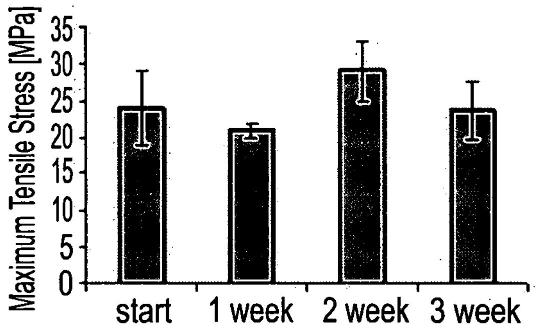


**FIG. 7C**

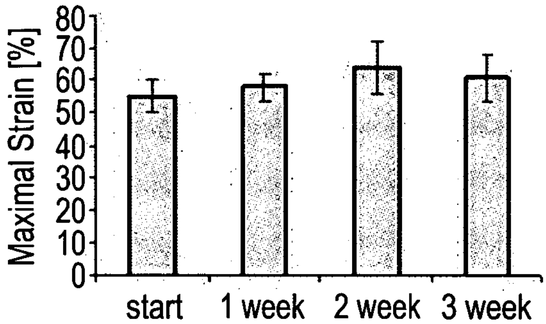


**FIG. 7D**

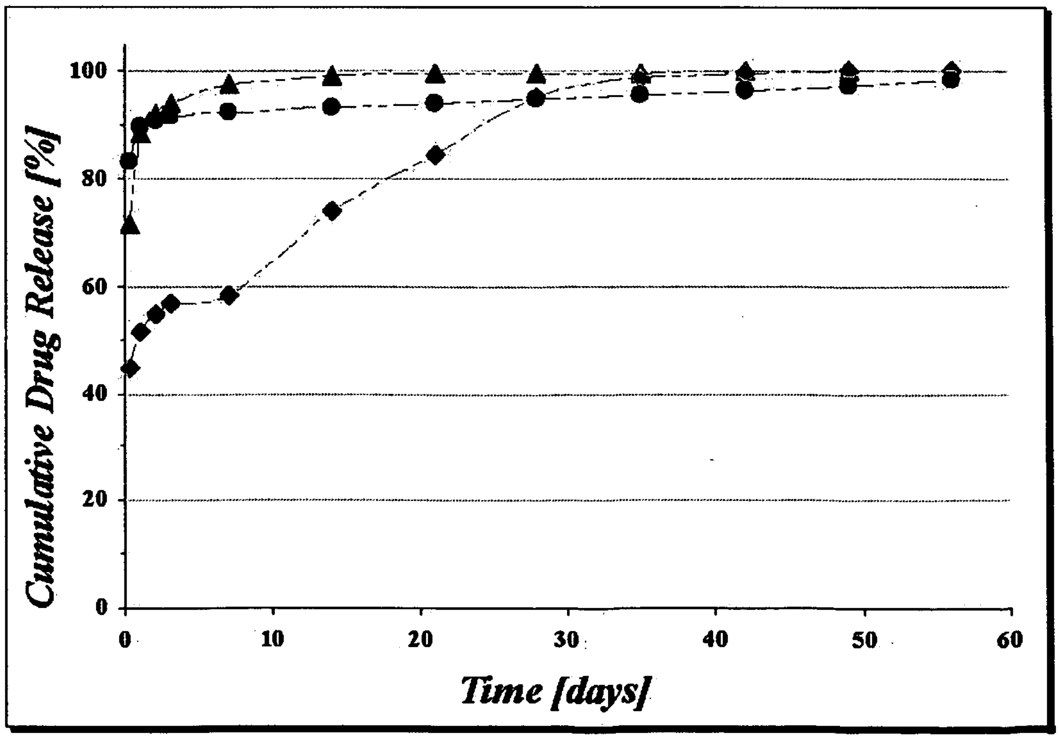


**FIG. 8**

FIG. 9

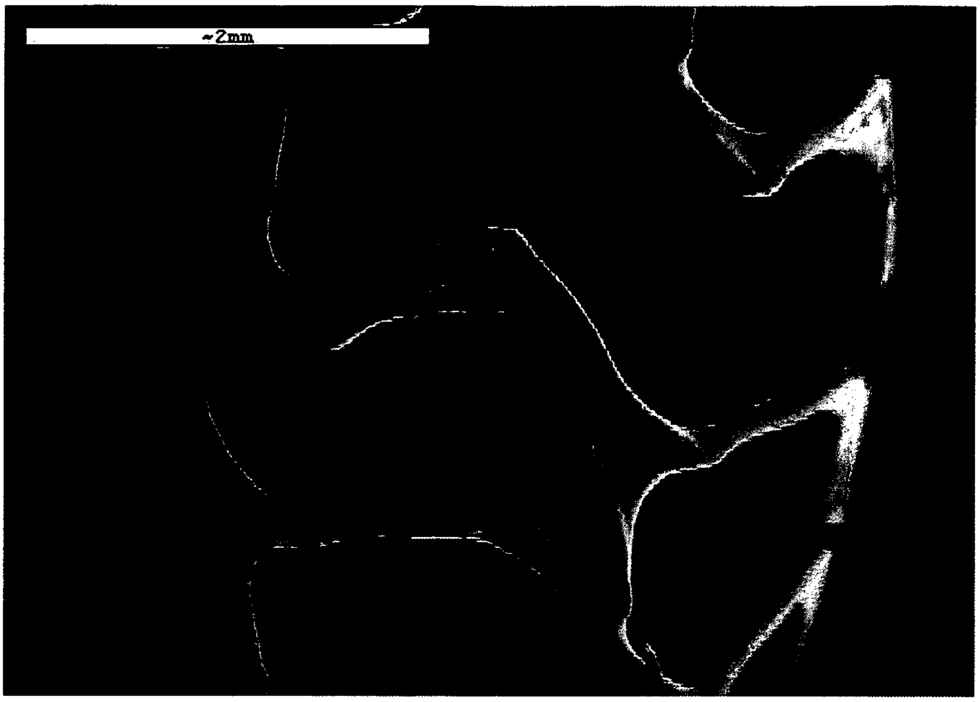


FIG. 10

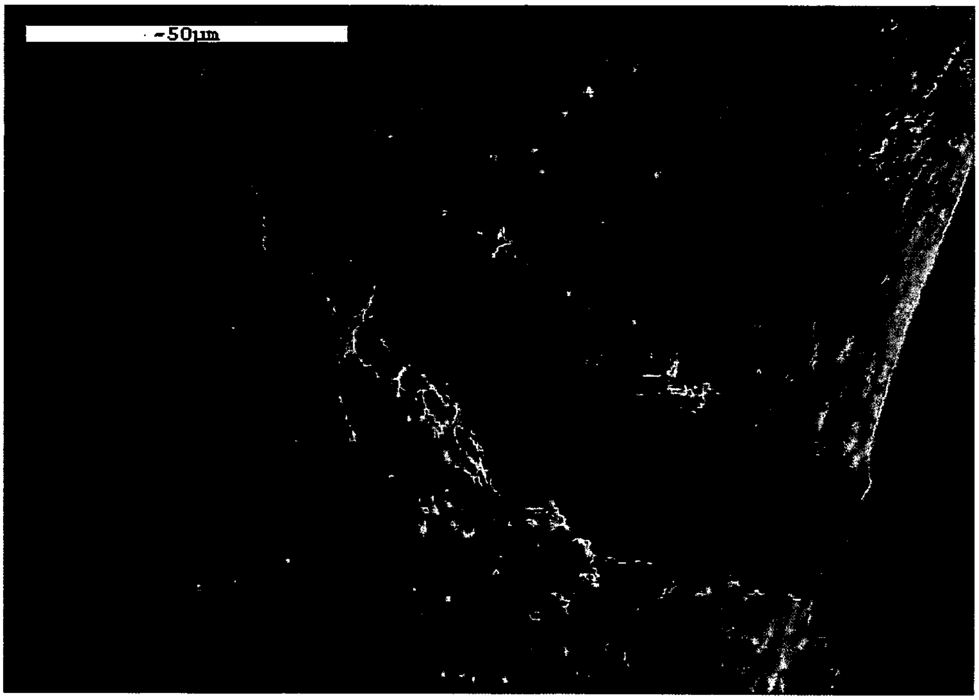

FIG. 11

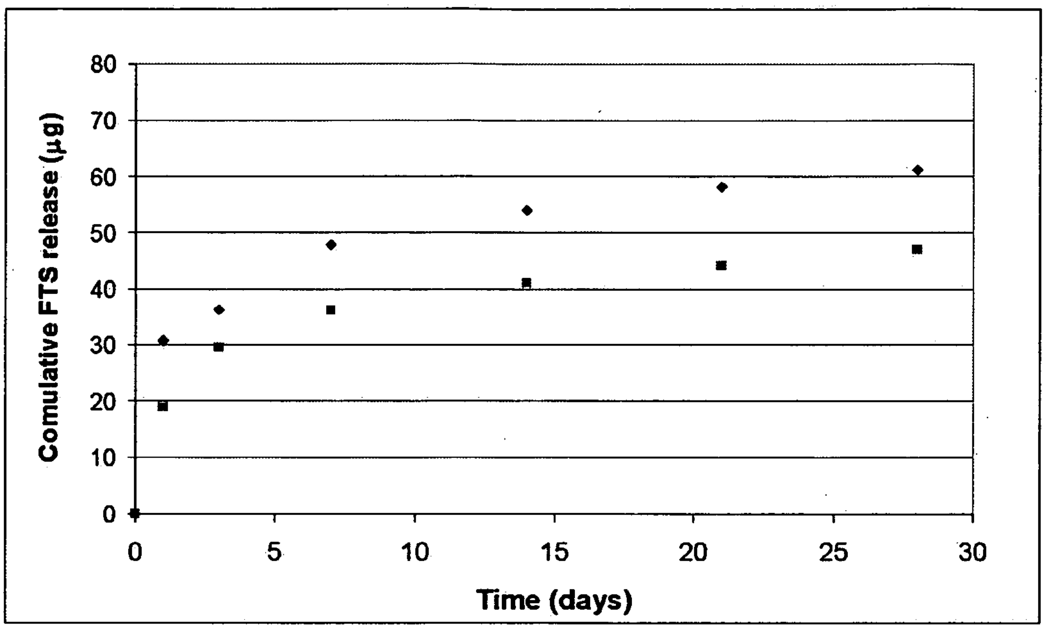
Comulative FTS release (µg)
80
70
60
50
40
30
20
10
0
0
5
10
15
20
25
30
Time (days)

FIG. 12

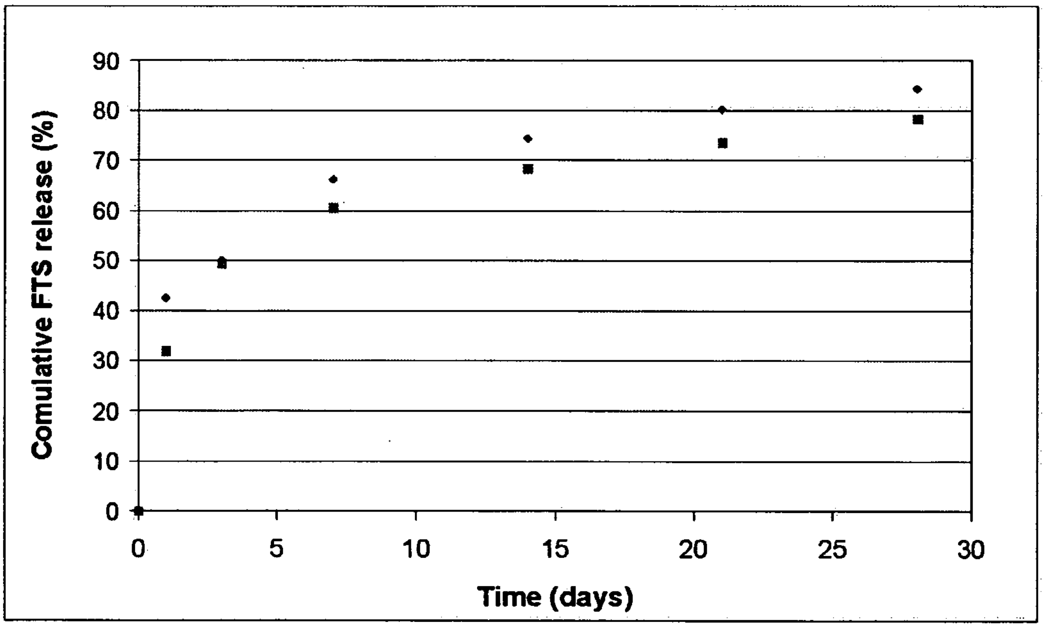
Comulative FTS release (%)
90
80
70
60
50
40
30
20
10
0
0
5
10
15
20
25
30
Time (days)

FIG. 13

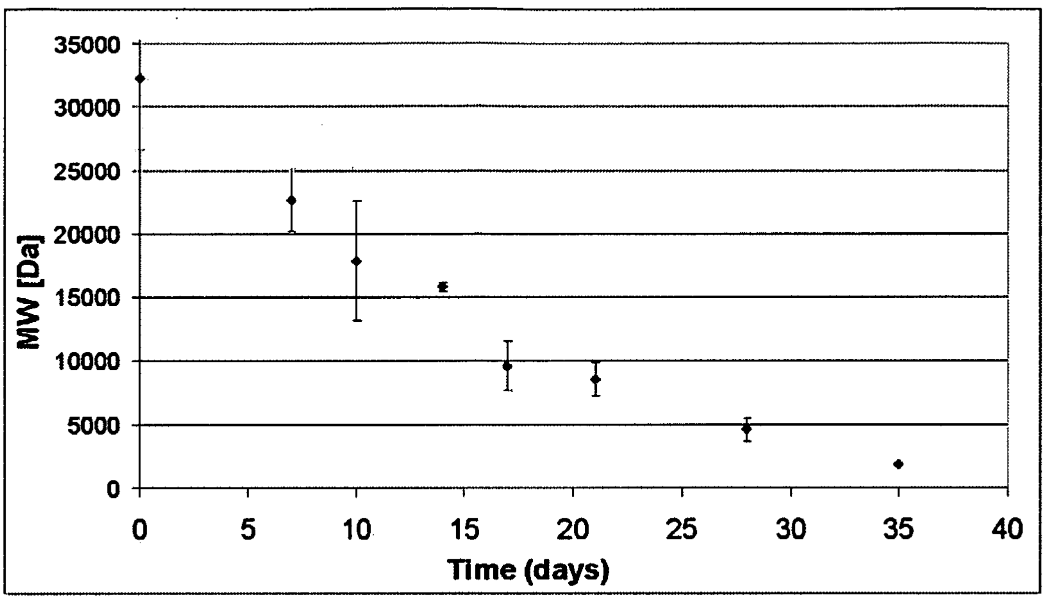
35000
30000
25000
20000
15000
10000
5000
0
MW [Da]
0
5
10
15
20
25
30
35
40
Time (days)

FIG. 14

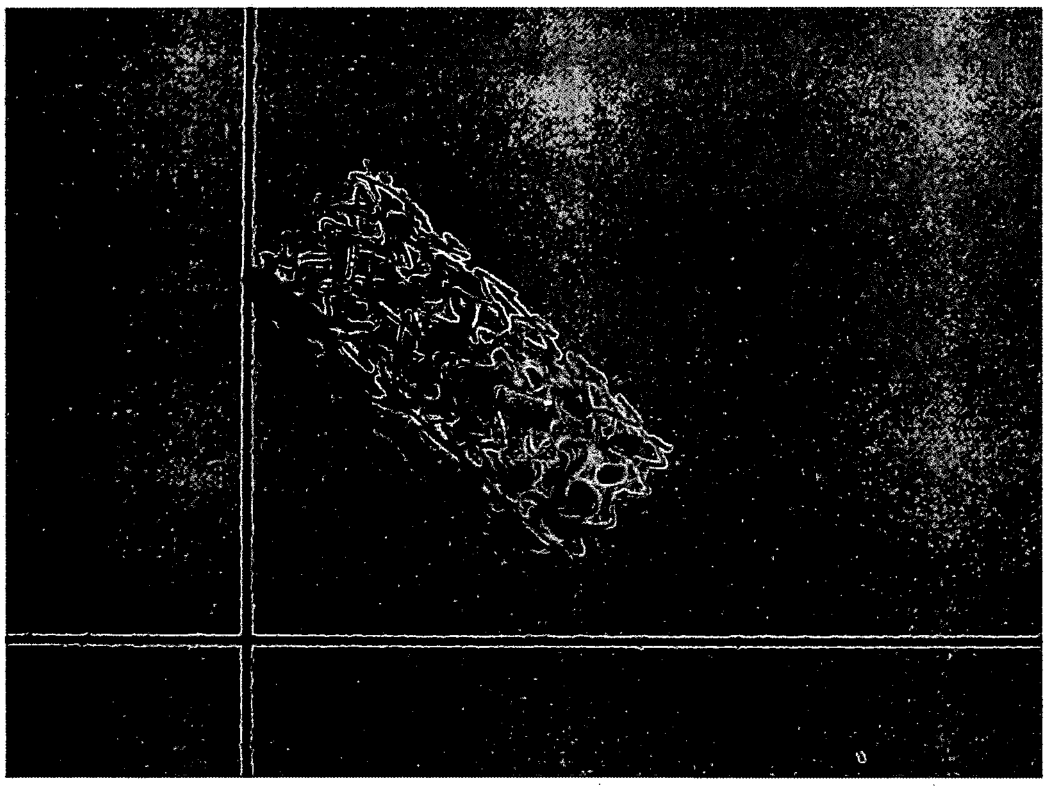

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5085629 A
- US 7169187 B
- US 20030050687 A
- US 20070134305 A
- US 5705528 A


### Non-patent literature cited in the description


- **GOLDBERG, L. et al.** *J. Med. Chem.,* 2009, vol. 52 (1), 197-205
- **MARCIANO, D. et al.** *J. Med. Chem.,* 1995, vol. 38 (8), 1267-1272
- **TAMAI, H. et al.** *Circulation,* 2000, vol. 102 (4), 399-404
- **TAMAI, H. et al.** *J. Intervent. Cardiol.,* 1999, vol. 12, 443-449
- **VIRMANI, R. et al.** *Circulation,* 2004, vol. 109 (6), 701-5
- **REVELL, P. A. et al.** *Biomaterials,* 1998, vol. 19 (17), 1579-86
- **SERRUYS, P. W. et al.** *J Am Coll Cardiol,* 2005, vol. 46 (2), 253-60
- **CARTER, A. J. et al.** *Cardiovasc Res,* 2004, vol. 63 (4), 617-24
- **WANG, X. et al.** *Biomaterials,* 2006, vol. 27 (32), 5588-95
- **VENKATRAMAN, S. et al.** *J Control Release,* 2007, vol. 120 (3), 149-60
- **COLOMBO, A. et al.** *Circulation,* 2003, vol. 108 (7), 788-94
- **DRACHMAN, D. E. et al.** *J Am Coll Cardiol,* 2000, vol. 36 (7), 2325-32
- **FINKELSTEIN, A. et al.** *Circulation,* 2003, vol. 107 (5), 777-84
- **WESTEDT, U. et al.** *J Control Release,* 2006, vol. 111 (1-2), 235-46
- **BREITENBACH, A. et al.** *J Control Release,* 2000, vol. 63 (1-2), 53-68
- **GALDBART, J. O. et al.** *J Surg Res,* 1996, vol. 66 (2), 174-8
- **STRACHAN, C. J. et al.** *Eur J Vasc Surg,* 1991, vol. 5 (6), 627-32